# EUROPEAN PATENT APPLICATION

(11) **EP 4 257 703 A1**
(43) Date of publication of application: **11.10.2023**
(21) Application number: 21903672.0
(22) Date of filing: 19.11.2021
(51) Int. Cl.: C12Q 1/6874, C12N 5/0775, A61K 35/28, A61P 21/00, A23L 33/18, A61K 48/00

(54) **METHOD FOR SELECTING MESENCHYMAL STEM CELLS HAVING IMPROVED SELF-MAINTENANCE ABILITY, AND MESENCHYMAL STEM CELLS SELECTED THEREBY**

(30) Priority: 07.12.2020 KR 20200169835
(71) Applicant: Samsung Life Public Welfare Foundation, Seoul 04348 (KR); Encell Co., Ltd., Gangnam-gu, Seoul 06072 (KR)
(72) Inventor: CHANG, Jong Wook, Seoul 06351 (KR); JEON, Hong Bae, Seoul 05531 (KR); PARK, Sang Eon, Seongnam-si, Gyeonggi-do 13500 (KR); KIM, Sun Jeong, Seoul 05696 (KR)
(74) Representative: Patentanwälte Olbricht Buchhold Keulertz
(86) International application number: PCT/KR2021/017083
(87) International publication number: WO 2022/124626

(57) **Abstract**

The present disclosure relates to a method for selecting mesenchymal stem cells having improved self-maintenance ability, wherein mesenchymal stem cells having excellent self-proliferative ability may be selected to reduce donor variation, and mesenchymal stem cells having excellent self-proliferative ability may be secured in large quantities and used for the development of cell therapy products.

## Description

### Technical Field

This application claims the benefit of Korean Patent Application No. 10-2020-0169835 filed on December 7, 2020, in the Korean Intellectual Property Office, the disclosure of which is incorporated herein in its entirety by reference.

The present disclosure relates to a method for selecting mesenchymal stem cells having improved self-maintenance ability.

### Background Art

Mesenchymal stem cells, as multipotent cells, are isolated from a variety of tissues such as bone marrow, adipose tissue, placenta, and umbilical cord blood and have the capacity to differentiate into bone, cartilage, muscle, fat, and fibroblasts. Extensive research has been conducted into the use of mesenchymal stem cells as a treatment for various diseases by regenerating non-regenerative tissues or cells by using such characteristics of stem cells. However, as a cell therapy product, mesenchymal stem cells have problems of large donor variation, low production yield, and high manufacturing costs, as well as failure to show long-term therapeutic effects due to a short survival period in the body.

Therefore, there is a need to select mesenchymal stem cells having high proliferative ability and high therapeutic efficacy to produce stem cells for development of stem cell therapy products.

### Disclosure

### Technical problem

One aspect is to provide a method for selecting mesenchymal stem cells having improved self-maintenance ability, the method including measuring an expression or activity level of a mesenchymal stem cell (MSC) self-maintenance factor (MSMF) after culturing mesenchymal stem cells.

Another aspect is to provide mesenchymal stem cells selected by the method.

Another aspect is to provide an apoptosis inhibitor including mesenchymal stem cells genetically engineered to reduce the expression of the MSMF or suppress the expression of the MSMF compared to parent cells, or including the MSMF.

Another aspect is to provide a pharmaceutical composition for preventing or treating muscle diseases including mesenchymal stem cells genetically engineered to secrete the MSMF or overexpress the MSMF compared to parent cells, or including the MSMF.

Another aspect is to provide a pharmaceutical composition for preventing or treating muscle diseases including the MSMF as an active ingredient.

Another aspect is to provide a health functional food composition for preventing or alleviating muscle diseases including the MSMF as an active ingredient.

Another aspect is to provide a cell therapy product including mesenchymal stem cells genetically engineered to secrete the MSMF or overexpress the MSMF compared to parent cells, or including the MSMF.

Another aspect is to provide a method for preventing or treating muscle diseases, the method including administering mesenchymal stem cells genetically engineered to secrete the MSMF or overexpress MSMF compared to parent cells, or a pharmaceutically effective dose MSMF, to an individual in need thereof.

Another aspect is to provide a use of a composition including mesenchymal stem cells genetically engineered to secrete MSMF or overexpress MSMF compared to parent cells, or MSMF for treating muscle diseases.

Another aspect is to provide a use of a composition including mesenchymal stem cells genetically engineered to secrete the MSMF or overexpress the MSMF compared to parent cells, or the MSMF for preparing a therapeutic agent for muscle diseases.

### Technical Solution

One aspect provides a method for selecting mesenchymal stem cells having improved self-maintenance ability, the method including measuring an expression or activity level of a mesenchymal stem cell (MSC) self-maintenance factor (MSMF) after culturing mesenchymal stem cells.

As used herein, the term "mesenchymal stem cells (MSCs)" refers to multipotent stem cells maintaining the ability to self-renew and stemness and having the ability to differentiate into various mesenchymal tissues such as mesodermal cells, e.g., bone, cartilage, fat, and muscle cells, and ectodermal cells, e.g., nerve cells. The mesenchymal stem cells may be derived from umbilical cord, umbilical cord blood, bone marrow, fat, muscle nerve, skin, amnion, chorion, decidua, placenta, and the like. In addition, the mesenchymal stem cells may be derived from humans, fetuses, or non-human mammals.

As used herein, the term "MSC self-maintenance factor (MSMF)" refers to various regulators secreted by mesenchymal stem cells, essential for self-maintenance of mesenchymal stem cells, and expressed in a time-specific and cell-specific manner. The MSMF may be several proteins or genes thereof involved in adhesion, differentiation, chemotaxis, or proliferation. The MSMF may be, for example, FBLN5, OLR1, TNFAIP6, ANXA3, IL6, POU2F2, TNFAIP2, SERPINE2, INHBA, VEGFA, HMGB1, CSF2, GATA3, PCSK6, SYN1, F2RL1, DOCK2, SLC9A4, STX1B, RARRES2, CXCL1, FGF7, PLAU, SCG2, NR4A3, COR01A, CHRM3, NPR3, BST2, GATA4, CREG1, FGF7, YPEL5, or AURKA. Specifically, proteins involved in adhesion may be FBLN5, OLR1, TNFAIP6, ANXA3, and the like, and proteins involved in differentiation may be IL6, POU2F2, TNFAIP2, SERPINE2, INHBA, VEGFA, HMGB1, CSF2, GATA3, PCSK6, SYN1, F2RL1, DOCK2, SLC9A4, STX1B, RARRES2, and the like. In addition, proteins involved in chemotaxis may be IL6, CXCL1, VEGFA, FGF7, PLAU, HMGB1, SCG2, NR4A3, GATA3, DOCK2, COR01A, RARRES2, and the like, and proteins involved in proliferation may be CXCL1, DOCK2, CHRM3, NPR3, NR4A3, F2RL1, BST2, GATA4, CREG1, FGF7, YPEL5, AURKA, and the like.

In one embodiment, the method includes: culturing the mesenchymal stem cells and treating the cultured mesenchymal stem cells with an expression or activity inhibitor of the MSMF; measuring an expression or activity level of the MSMF in the mesenchymal stem cells in which expression or activity of the MSMF is inhibited; and separating mesenchymal stem cells in which the expression or activity level of the MSMF is reduced compared to a control group untreated with the expression or activity inhibitor of the MSMF.

The expression or activity inhibitor of the MSMF may be selected from the group consisting of small interference RNA (siRNA), short hairpin RNA (shRNA), microRNA (miRNA), ribozyme, DNAzyme, peptide nucleic acids (PNA), antisense oligonucleotides, antibodies, aptamers, and compounds and natural extracts directly binding to the MSMF protein and inhibiting the activity thereof. In one embodiment, the expression or activity inhibitor of the MSMF may be a first primer set represented by SEQ ID NOS: 5 and 6 and a second primer set represented by SEQ ID NOS: 9 and 10.

The measuring of the expression or activity level of the MSMF may be performed by a method selected from the group consisting of reverse transcription polymerase chain reaction, competitive reverse transcription polymerase chain reaction, real-time reverse transcription polymerase chain reaction, RNase protection assay, Northern blotting, and DNA chip assay. In addition, the method may be selected from the group consisting of protein chip analysis, immunoassay, ligand binding assay, matrix desorption/ionization time of flight mass spectrometry (MALDI-TOF) assay, surface enhanced laser desorption/ionization time of flight mass spectrometry (SELDI-TOF) assay, radioimmunoassay, radial immunodiffusion assay, Ouchterlony immunodiffusion assay, rocket immunoelectrophoresis, immunohistostaining, complement fixation assay, two-dimensional electrophoresis, liquid chromatography-mass spectrometry (LC-MS), liquid chromatography-mass spectrometry/mass spectrometry (LC-MS/MS), Western blotting, and enzyme linked immunosorbent assay (ELISA).

Mesenchymal stem cells having improved self-maintenance ability in a medium may be selected by isolating mesenchymal stem cells in which the expression or activity level of the MSMF is reduced compared to a control untreated with the expression or activity inhibitor of the MSMF. In one embodiment, it was confirmed that migration ability, colony formation ability, and cell proliferative ability of mesenchymal stem cells decreased as the expression of the MSMF decreased. Also, it was confirmed that a doubling time of the mesenchymal stem cells increased. Therefore, since expression or activity of the MSMF is increased in the mesenchymal stem cells with improved self-maintenance ability, desired cells may be obtained by isolating the mesenchymal stem cells in which the expression or activity of the MSMF is maintained or reduced. According to the method, mesenchymal stem cells may be mass-produced as well as manufacturing costs may be reduced by selecting mesenchymal stem cells having high proliferative ability in the early stage.

Another aspect provides mesenchymal stem cells selected by the above-described method. Another aspect provides an apoptosis inhibitor including mesenchymal stem cells genetically engineered to reduce the expression of the MSMF or suppress the expression of the MSMF compared to parent cells, or including the MSMF. Another aspect provides a method for inhibiting apoptosis, the method including: brining the mesenchymal stem cells genetically engineered to reduce the expression of the MSMF or suppress the expression of the MSMF compared to parent cells and/or the MSMF into contact with cells *in vitro,* or administering the genetically engineered mesenchymal stem cells or MSMF to an experimental animal *in vivo.* The mesenchymal stem cells may have an increased expression level of the MSMF that is essential for self-maintenance. That is, the mesenchymal stem cells may have improved stemness, migration ability, colony formation ability, and/or cell proliferative ability. In addition, the mesenchymal stem cells may have a reduced doubling time. That is, MSMF expression and cell doubling time may exhibit a negative correlation.

As used herein, the term "genetic engineering" or "genetically engineered" refers to the act of introducing one or more genetic modifications to a cell or a cell produced thereby. For example, the mesenchymal stem cells or host cells may be those genetically engineered to increase the expression or activity of the MSMF or an active fragment thereof, for example, those containing an exogenous gene encoding the MSMF or the active fragment thereof. The increased activity may mean that the activity of a protein or enzyme of the same type is higher than the activity of an endogenous protein or enzyme possessed or not possessed by parent cells, which are not genetically engineered (e.g., wild-type). The exogenous gene may be expressed in an amount sufficient to increase the activity of the above-mentioned protein in the mesenchymal stem cells or host cells compared to the parent cells. The exogenous gene may be introduced into parental cells via an expression vector. In addition, the exogenous gene may be introduced into the parent cells in the form of a linear polynucleotide. In addition, the exogenous gene may be expressed from an expression vector (e.g., plasmid) in a cell. In addition, the exogenous gene may be expressed in a form inserted into a genetic material (e.g., chromosome) in a cell for stable expression.

Another aspect provides a pharmaceutical composition for preventing or treating muscle diseases including mesenchymal stem cells genetically engineered to reduce the expression of the MSMF or suppress the expression of the MSMF compared to parent cells, or including the MSMF. Another aspect provides a method for preventing or treating muscle diseases, the method including administering the mesenchymal stem cells and/or MSMF to an individual. The mesenchymal stem cells genetically engineered to secrete the MSMF or overexpress the MSMF compared to parental cells or the MSMF are as described above. In one embodiment, it was confirmed that apoptosis was inhibited and cell proliferation was promoted by co-culturing muscle cells in which apoptosis was induced and mesenchymal stem cells treated with siAURKA and siDOCK2. Therefore, mRNA of AURKA and DCOCK2 and proteins thereof may be used to prevent or treat muscle diseases by inhibiting apoptosis of muscle cells.

The muscle disease may be, for example, Charcot-Marie-Tooth disease, Spinal Muscular Atrophy (SMA), Lou Gehrig's disease (amyotrophic lateral sclerosis, ALS), Duchenne Muscular Dystrophy, Myotonic Dystrophy, sarcopenia, muscular atrophy, myasthenia, muscular dystrophy, myotonia, hypotonia, muscular weakness, muscular dystrophy, amyotrophic lateral sclerosis, or inflammatory myopathy.

Another aspect provides a pharmaceutical composition for preventing or treating muscle diseases including the MSMF as an active ingredient. In one embodiment, the MSMF may be isolated from mesenchymal stem cells.

In another embodiment, the composition may further include mesenchymal stem cells. The mesenchymal stem cells may secrete the MSMF or an active fragment thereof or may be genetically engineered to secrete the MSMF or the active fragment thereof. Specifically, the mesenchymal stem cells may be those modified by insertion or injection of DNA in cell culture by a method of modifying, enhancing, or supplementing cell functions for induction for structural or therapeutic purposes. Therefore, the MSMF and mesenchymal stem cells may be co-administered.

The pharmaceutical composition for preventing or treating muscle diseases according to an aspect may be formulated into various forms such as oral formulations such as powders, granules, tablets, capsules, suspensions, emulsions, syrups, and aerosols, formulations for external use, suppositories, and sterile injection solutions, and may include an appropriate carrier, excipient, or diluent commonly used for formulation in preparation of pharmaceutical compositions.

The carrier, excipient, or diluent may be various compounds or mixtures including lactose, dextrose, sucrose, sorbitol, mannitol, xylitol, erythritol, maltitol, starch, Acacia gum, alginate, gelatin, calcium phosphate, calcium silicate, cellulose, methyl cellulose, amorphous cellulose, polyvinyl pyrrolidone, water, methylhydroxybenzoate, propylhydroxybenzoate, talc, magnesium stearate, and mineral oil.

The pharmaceutical composition may be formulated using a diluent or excipient commonly used in the art such as a filler, an extender, a binder, a humectant, a disintegrant, and a surfactant.

Solid formulations for oral administration may be prepared by mixing a legume extract with at least one excipient such as starch, calcium carbonate, sucrose or lactose, and gelatin. In addition to simple excipients, a lubricant such as magnesium stearate and talc may also be used.

Liquid formulations for oral administration, which are suspensions, formulations for internal use, emulsions, and syrups, may include various excipients such as a humectant, a sweetener, a flavoring agent, and a preservative, in addition to simple diluents such as water and liquid paraffin.

Formulations for parenteral administration include sterile aqueous solutions, nonaqueous solvent, suspensions, emulsions, lyophilizates, and suppositories. As the nonaqueous solvents and suspensions, propylene glycol, polyethylene glycol, vegetable oil such as olive oil, injectable ester such as ethyl oleate, and the like may be used. As a base material for suppositories, witepsol, macrogol, tween 61, cacao butter, laurin butter, glycerolgelatin, and the like may be used.

A preferred dosage of the pharmaceutical composition for preventing or treating muscle diseases according to one aspect varies according to the condition and body weight of a patient, severity of disease, form of drug, route and duration of administration, but may be appropriately selected by those skilled in the art. However, for desirable effects, the dosage may be from 0.0001 to 2,000 mg/kg, preferably, from 0.001 to 2,000 mg/kg per day. The pharmaceutical composition may be administered once a day or several times a day at divided doses. However, the scope of the present invention is not limited by the dosage.

The pharmaceutical composition for preventing or treating muscle diseases according to one aspect may be administered to mammals such as rats, mice, livestock, and humans via various routes. Administration may be conducted via any method, for example, orally or rectally, or by intravenous, muscular, subcutaneous, intrauterine subdural, or intracerebroventricular injection.

Another aspect provides a health functional food composition for preventing or alleviating muscle diseases including mesenchymal stem cells genetically engineered to reduce the expression of the MSMF or suppress the expression of the MSMF compared to parent cells, or including the MSMF. The mesenchymal stem cells or the MSMF are as described above.

In the health functional food for preventing or alleviating muscle diseases according to one aspect, when the compound is used as an additive to the health functional food, it may be added as it is or used together with other foods or food ingredients and may be appropriately used according to any method commonly used in the art. The amount of the active ingredient to be mixed therewith may be appropriately determined according to the intended use, e.g., prevention, health, or treatment.

The health functional food may be provided in the form of all food or drink types commonly used in the art as well as in the form of powders, granules, pills, tablets, and capsules.

The type of the food is not particularly limited, and examples of the food to which the substance may be added, may include meats, sausages, breads, chocolates, candies, snacks, cookies, pizzas, ramens, other noodles, gums, dairy products including ice cream, various kinds of soups, beverages, teas, drinks, alcoholic beverages, and vitamin complexes, and may also include all foods that are considered within conventional meaning.

In general, in the preparation of foods or drinks, the compound may be added in an amount of 15 parts by weight or less, preferably 10 parts by weight or less, based on 100 parts by weight of raw materials. However, in the case of a long-term intake for the purposes of health and hygiene or health control, the amount may be less than the above-described range. In addition, there is no problem in terms of safety since fractions from natural substances are used, and thus an amount greater than the above-described range may also be used.

Among health functional foods according to one aspect, drinks, like conventional drinks, may further include various flavoring agents or natural carbohydrates as additional ingredients. Examples of the above-described natural carbohydrates may include monosaccharides such as glucose and fructose, disaccharides such as maltose and sucrose, polysaccharides such as dextrin and cyclodextrin, and sugar alcohols such as xylitol, sorbitol and erythritol. Examples of the sweetener may include natural sweeteners such as thaumatin and stevia extract and synthetic sweeteners such as saccharin and aspartame. The ratio of the natural carbohydrate may be from about 0.01 to 0.04 g, preferably from about 0.02 to 0.03 g per 100 mL of the drink according to the present invention.

The health functional food for preventing or alleviating muscle diseases according to one aspect may further include various nutrients, vitamins, electrolytes, flavors, colorants, pectic acid and salts thereof, alginic acid and salts thereof, organic acids, protective colloidal thickeners, and pH adjusters, stabilizers, preservatives, glycerin, alcohols, carbonating agents used in carbonated drinks. In addition, the composition for improving sleep of the present disclosure may include fruit flesh for production of natural fruit juice, fruit juice-flavored drinks, and vegetable-based beverages. These ingredients may be used independently or in combination. The ratio of these additives is not limited, but is generally selected from the range of 0.01 to 0.1 parts by weight based on 100 parts by weight of the health functional food of the present disclosure.

Another aspect provides a cell therapy product including the mesenchymal stem cells as an active ingredient. The mesenchymal stem cells are as described above.

As used herein, the term "cell therapy product" refers to cells and tissues prepared by isolation from an individual, culture, and specific manipulation and used as a medicament for the purpose of treatment, diagnosis, and prevention of a disease (FDA regulations, USA) and means a medicament used for the purpose of treatment, diagnosis, and prevention of a disease through a series of actions such as proliferation and selection of living autologous, allogenic, or xenogenic cells *in vitro* or changes of biological properties of cells by different methods, in order to restore the functions of cells or tissues. In addition, the "treatment" refers to all actions involved in alleviating or beneficially changing symptoms of a disease by administering a cell therapy product. The mesenchymal stem cells may be those having increased expression of the MSMF, and expression of FBLN5, TNFAIP6, ANXA3, IL6, POU2F2, TNFAIP2, INHBA, VEGFA, CSF2, GATA3, CXCL1, HMGB1, BST2, and AURKA associated with inflammatory diseases, immune diseases, or cancer may be increased. Therefore, in one embodiment, the cell therapy product may be used to treat muscle diseases, inflammatory diseases, immune diseases, or cancer.

Examples of the inflammatory disease may include atopy, psoriasis, dermatitis, allergy, arthritis, rhinitis, otitis media, sore throat, tonsillitis, cystitis, nephritis, pelvic inflammatory disease, Crohn's disease, ulcerative colitis, ankylosing spondylitis, systemic lupus erythematosus (SLE), asthma, edema, delayed allergy (type IV allergy), graft rejection, graft versus host disease, autoimmune encephalomyelitis, multiple sclerosis, inflammatory bowel disease, cystic fibrosis, diabetic retinopathy, ischemic-reperfusion injury, vascular restenosis, glomerulonephritis, and gastrointestinal allergy.

Examples of the immune disease may include autoimmune diseases such as rheumatoid arthritis, type I diabetes, multiple sclerosis, systemic lupus erythematosus, and inflammatory diseases such as asthma, encephalitis, inflammatory enteritis, chronic obstructive pulmonary disease, allergy, septic shock, pulmonary fibrosis, undifferentiated spondyloarthrosis, undifferentiated arthropathy, arthritis, inflammatory osteolysis, and chronic inflammation caused by chronic viral or bacterial infection.

Examples of the cancer may include multiple myeloma, lung cancer, liver cancer, gastric cancer, colorectal cancer, colon cancer, skin cancer, bladder cancer, prostate cancer, breast cancer, ovarian cancer, cervical cancer, thyroid cancer, renal cancer, fibrosarcoma, melanoma, and hematologic malignancy.

The cell therapy product may be administered after being prepared into a pharmaceutical formulation in a unit dosage form suitable for administration into a patient's body according to any common method in the pharmaceutical field, and the formulation may include an effective amount for a single dose or for divided doses. As a formulation suitable for this purpose, a formulation for parenteral administration such as an injection formulation, e.g., an ampoule for injection, an infusion formulation, e.g., an infusion bag, and a spray formulation, e.g., an aerosol may be used. The ampoule for injection may be mixed with an injectable solution immediately before use, and physiological saline, glucose, mannitol, Ringer's solution, or the like may be used as the injectable solution. In addition, the infusion bag may be made of polyvinyl chloride or polyethylene and those manufactured by Baxter, Becton Dickinson, Medcep, National Hospital Products, or Terumo corporations may be used.

The pharmaceutical formulation may further include at least one pharmaceutically acceptable inert carrier, e.g., a preservative, an analgesic, a solubilizer, or a stabilizer for injectable formulations and an excipient, a lubricant, or a preservative for topical formulations.

The cell therapy product or pharmaceutical preparation of the present disclosure may be administered by a common administration method used in the art, together with other stem cells used for transplantation and other purposes, in the form of a mixture therewith. Direct engraftment or transplantation to a lesion of a patient in need of treatment, or direct transplantation or injection into the peritoneal cavity is preferred, without being limited thereto. Furthermore, both of a non-surgical administration using a catheter and a surgical administration such as injection or transplantation after incision are possible, but non-surgical administration using a catheter is more preferred. In addition, parenteral administration according to a common method, for example, transplantation by infusion into the blood vessel as a common method is also possible as well as direct administration into a lesion.

The daily dose of the mesenchymal stem cells may be 1.0×10⁴ to 1.0×10¹⁰ cells/kg (body weight), preferably 2.5×10⁵ to 5×10⁷ cells/kg (body weight) and may be administered once or as several divided doses. However, it should be understood that the actual dose of active ingredients is determined in consideration of various related factors such as a disease to be treated, severity of the disease, route of administration, body weight, age and gender of a patient, and therefore the dose should not be construed as limiting the scope of the present disclosure in any manner.

The mesenchymal stem cells may be used in various types of treatment protocols for enhancement, treatment, or replacement of tissues or organs of the body by engraftment, transplantation, or infusion of desired cell populations.

The cell therapy product may be used in an unfrozen state or frozen for future use. In the case of being frozen, a standard cryopreservative (e.g., DMSO, glycerol, and Epilife^{®} cell freezing medium (Cascade Biologics)) may be added to cell populations before being frozen.

As described above, the mesenchymal stem cells or the MSMF according to one aspect may be used as a preventive or therapeutic agent for various muscle diseases such as sprains, contusions, and spasm by inhibiting apoptosis of muscle cells.

### Advantageous Effects

According to the method of the present disclosure, donor variation may be reduced by selecting mesenchymal stem cells having excellent self-proliferative ability, and mesenchymal stem cells may be mass-produced, and thus the method may be used for development of cell therapy products. In addition, the mesenchymal stem cells selected according to the above-described method has an increased survival period in *vivo,* thereby increasing therapeutic effects.

### Description of Drawings

FIG. 1A is a graph showing doubling time of a P-high group and a P-low group.
FIG. 1B shows results of comparison of expression levels of genes involved in self-maintenance in the P-low group and the P-high group.
FIG. 1C is a graph showing comparison of expression levels of AURKA and DOCK2, which are selected as MSMF gene candidates, in the P-low group and the P-high group.
FIG. 2A is a graph showing comparison of relative mRNA expression levels of AURKA and DOCK2 by selected siRNA sequences.
FIG. 2B shows protein expression levels of AURKA and DOCK2 after knocking down an MSMF gene by transfecting mesenchymal stem cells with siAURKA and siDOCK2.
FIG. 2C is a graph showing stemness identified by FACS after knocking down an MSMF gene by transfecting mesenchymal stem cells with siAURKA and siDOCK2.
FIG. 3A shows migration ability of mesenchymal stem cells after knocking down an MSMF gene by transfecting mesenchymal stem cells with siAURKA and siDOCK2.
FIG. 3B is a graph showing whether mesenchymal stem cells form colonies after knocking down an MSMF gene by transfecting the mesenchymal stem cells with siAURKA and siDOCK2.
FIG. 3C is a graph showing doubling time of mesenchymal stem cells after knocking down an MSMF gene by transfecting the mesenchymal stem cells with siAURKA and siDOCK2.
FIG. 3D shows cell proliferative ability of mesenchymal stem cells after knocking down an MSMF gene by transfecting the mesenchymal stem cells with siAURKA and siDOCK2.
FIGS. 3E and 3F show effects of inhibition of expression of AURKA and DOCK2 genes on phosphorylation of AKT and ERK, which are kinases involved in proliferation of cells, and phosphorylation of FAK and JNK, which are kinases involved in migration ability.
FIG. 4A is a graph showing comparison of relative mRNA expression levels of AURKA and DOCK2 in 10 lots of mesenchymal stem cells.
FIG. 4B is a graph sequentially showing doubling time in 10 lots of mesenchymal stem cells.
FIG. 4C shows graphs of correlation between mRNA expression levels of AURKA and DOCK2 and doubling time.
FIG. 4D shows protein expression levels of AURKA and DOCK2 in mesenchymal stem cells.
FIGS. 4E and 4F show results of confirming phosphorylation levels of AKT, ERK, FAK, and JNK in mesenchymal stem cells.
FIG. 5A shows results of identifying expression of apoptosis-related proteins according to mRNA expression levels of AURKA and DOCK2, by co-culturing mesenchymal stem cells and muscle cells in which apoptosis is induced.
FIG. 5B shows results of identifying degrees of reduction in apoptosis according to mRNA expression levels of AURKA and DOCK2, by co-culturing mesenchymal stem cells and muscle cells in which apoptosis is induced.
FIG. 6A shows results of confirming expression of apoptosis-related proteins according to inhibition of expression of AURKA and DOCK2 genes, by co-culturing mesenchymal stem cells in which AURKA and DOCK2 genes are knocked down and muscle cells in which apoptosis is induced.
FIG. 6B shows results of identifying degrees of reduction in apoptosis according to inhibition of expression of AURKA and DOCK2 genes, by co-culturing mesenchymal stem cells in which AURKA and DOCK2 genes are knocked down and muscle cells in which apoptosis is induced.
FIG. 6C shows protein expression levels of XCL1 of mesenchymal stem cells in which an AURKA gene is knocked down.
FIG. 7A shows inhibitory effects on apoptosis of muscle tissue according to a difference in mRNA expression levels of AURKA of mesenchymal stem cells in a muscular dystrophy mouse model.
FIG. 7B shows differences in inhibitory effects on apoptosis of muscle tissue according to differences in mRNA expression levels of AURKA of mesenchymal stem cells in a muscular dystrophy mouse model.
FIG. 8A is a graph showing the degree of cell survival according to mRNA expression levels of AURKA of mesenchymal stem cells in a muscular dystrophy mouse model.
FIG. 8B shows inhibitory effects on fibrosis of muscle tissue according to mRNA expression levels of AURKA of mesenchymal stem cells in a muscular dystrophy mouse model.
FIG. 8C shows degrees of fibrosis of muscle tissue according to mRNA expression levels of AURKA of mesenchymal stem cells in a muscular dystrophy mouse model.

### Mode for Invention

Hereinafter, the present disclosure will be described in more detail with reference to the following examples. However, the following examples are merely presented to exemplify the present disclosure, and the scope of the present disclosure is not limited thereto.

### [Examples]

### Example 1. Isolation and Culture of Mesenchymal Stem Cells

All samples were collected with informed consent of donors under approval (IRB No. 2011-10-134) from the Institutional Review Board (IRB) of Samsung Medical Center. Mesenchymal stem cells were isolated by a conventionally known method. The isolated cells were aliquoted into a minimum essential medium (MEA) alpha (Invitrogen-Gibco, Rockville, MD) supplemented with 10% fetal bovine serum (FBS, Invitrogen-Gibco) and 50 µg/mL gentamycin (Invitrogen-Gibco) at a density of 3 ×10³ /cm² and incubated at 37°C under a 5% CO₂ condition.

### Example 2. Identification of Morphology and Doubling Time of Mesenchymal Stem Cells and Analytic Comparison of Gene Expression

The mesenchymal stem cells isolated and cultured in Example 1 were classified into mesenchymal stem cells satisfying the criteria of Good Manufacturing Practice (GMP) of Samsung Medical Center and mesenchymal stem cells not satisfying the criteria. As a result of measuring the doubling time of each of the mesenchymal stem cells classified according to the above criteria, it was confirmed that the mesenchymal stem cells satisfying the criteria had a shorter doubling time than that of the mesenchymal stem cells not satisfying the criteria. Based on these results, the cells were classified into a proliferation-high (P-high) group and a proliferation-low (P-low) group according to the doubling time, and the doubling time thereof was identified. Subsequently, genes expressed at high levels in the P-high group compared to the P-low group were compared and analyzed. Specifically, after extracting each RNA from the P-low group and the P-high group, RNA quality was measured using an Agilent 2100 Bioanalyzer and migration and peak patterns were analyzed. A microarray was performed using an Illumina Hiseq 2500 to compare and analyze genes whose log2 fold change was three times higher in the P-high group than in the P-low group.

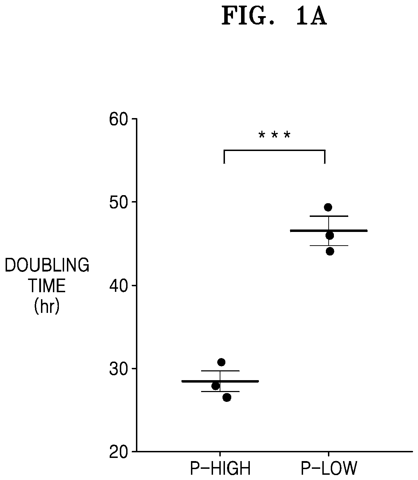

FIG. 1A is a graph showing doubling time of a P-high group and a P-low group.

As a result, as shown FIG. 1A, while the doubling time of the mesenchymal stem cells of the P-high group was 28.4 ± 1.2 hours, the doubling time of the mesenchymal stem cells of the P-low group was 47.9 ± 1.6 hours, which was 1.6 times longer than that of the P-high group.

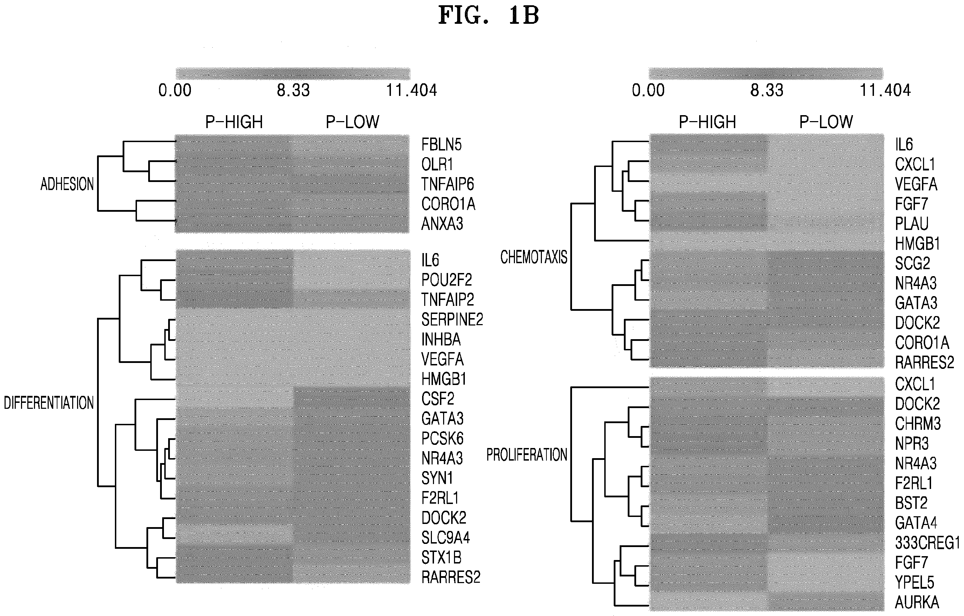

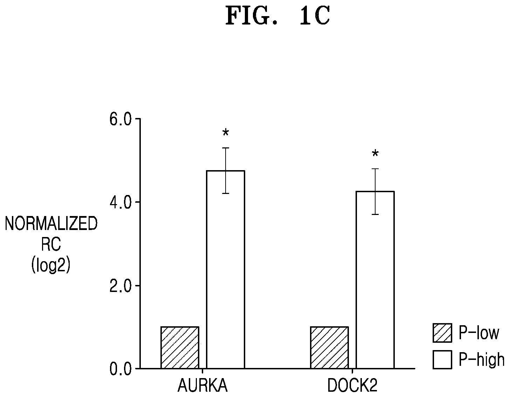

FIG. 1B shows results of comparison of expression levels of genes involved in self-maintenance in the P-low group and the P-high group. Green color represents genes exhibiting lower expression levels in the P-high group compared to the P-low group, and red color represents genes exhibiting higher expression levels in the P-high group compared to the P-low group. FIG. 1C is a graph showing comparison of expression levels of AURKA and DOCK2, which are selected as MSMF gene candidates, in the P-low group and the P-high group.

As a result, as shown in FIG. 1B, among the genes with a three-fold or more difference in the average log2 fold change values in the P-high group compared to the P-low group, 33 genes involved in adhesion, differentiation, chemotaxis, and proliferation were confirmed. Particularly, among the 33 genes, CORO1A, STX1B, HMGB1, DOCK2, AURKA, SLC9A4, CHRM3, NPR3, RARRES2, and ANXA3 were confirmed as genes expressed at higher levels in the P-high group compared to the P-low group. In addition, as shown in FIG. 1c, it was confirmed that AURKA and DOCK2 were expressed at about 4 times higher levels in the P-high group compared to the P-low group indicating a statistically significant difference. Therefore,
based on the above-described results, AURKA and DOCK2, which exhibited a statistically significant difference in the expression levels of the genes involved in adhesion, differentiation, chemotaxis, or proliferation, were selected as final candidates of the MSMF gene.

### Example 3. Selection of Optimal Sequence by Knock-down Efficacy Verification

### 3-1. Selection of siRNA Sequence Candidate for AURKA and DOCK2 and Identification of mRNA Expression Level

After verifying knock-down efficacy using siRNA libraries of AURKA and DOCK2 selected as the MSMF genes in Example 2, an optimal sequence was selected. Specifically, mesenchymal stem cells cultured in a growth medium by about 50% were transferred to a serum-free medium and transfected with three candidate sequences (Bioneer Corporation) for each of AURKA and DOCK2 obtained from the siRNA libraries (see Table 1 below) using a Lipofectamine RNAiMax (Invitrogen) at a concentration of 25 nM. After 48 hours, RNA was extracted from mesenchymal stem cells using an AccuPrep^{®} Universal RNA Extraction Kit and qRT-PCR was performed using a 2X Power SYBR Green Master Mix (AB). Subsequently, relative mRNA expression levels were compared with each other and a sequence exhibiting the greatest knock-down effect was selected as an optimal sequence for inhibiting expression of AURKA and DOCK2.

**[Table 1]**

| | | Sequence number | siRNA sequences |
|---|---|---|---|
| human AURKA | Candidate 1 | SEQ ID NO: 1 | GAGUCAUAGCAUGUGUGUA |
| | | SEQ ID NO: 2 | UACACACAUGCUAUGACUC |
| | Candidate 2 | SEQ ID NO: 3 | CUGUCAUUCGAAGAGAGUU |
| | | SEQ ID NO: 4 | AACUCUCUUCGAAUGACAG |
| | Candidate 3 | SEQ ID NO: 5 | GUGCAAUAACCUUCCUAGU |
| | | SEQ ID NO: 6 | ACUAGGAAGGUUAUUGCAC |
| human DOCK2 | candidate 1 | SEQ ID NO: 7 | CAGAACAAAAUCUGCUUCA |
| | | SEQ ID NO: 8 | UGAAGCAGAUUUUGUUCUG |
| | Candidate 2 | SEQ ID NO: 9 | CUGAGAAUGACUUCCUAC |
| | | SEQ ID NO: 10 | UGUAGGAAGUCAUUCUCAG |
| | Candidate 3 | SEQ ID NO: 11 | CAGAUGAGAGCAAAGACAA |
| | | SEQ ID NO: 12 | UUGUCUUUGCUCUCAUCUG |

As a result, it was confirmed that the sequence of Candidate 3 had the greatest knock-down effect in the case of siAURKA and the sequence of Candidate 2 had the greatest knock-down effect in the case of siDOCK2.

Therefore, after transfecting the mesenchymal stem cells with the sequence of Candidate 3 of siAURKA and the sequence of Candidate 2 of siDOCK2, sequence specificity was identified by comparing relative mRNA expression levels of AURKA and DOCK2 by the selected candidates.

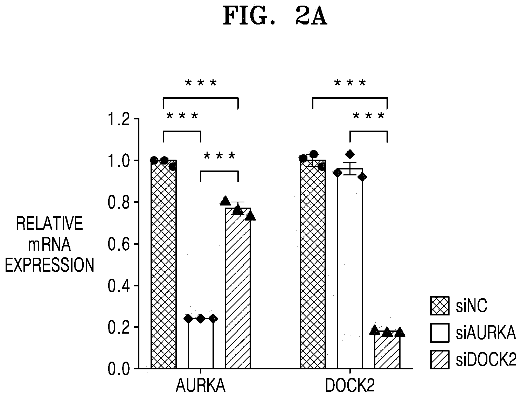

FIG. 2A is a graph showing comparison of relative expression levels of AURKA and DOCK2 by selected siRNA sequence.

As a result, as shown in FIG. 2A, in the case of AURKA, the mRNA expression was significantly reduced in the siAURKA-treated group compared to the siNC and siDOCK2-treated groups, and in the case of DOCK2, the mRNA expression was significantly reduced in siDOCK2-treated group compared to the siNC and siAURKA-treated groups. That is, the selected siRNA sequences may have sequence specificity for each MSAF gene.

### 3-2. Identification of Protein Expression Level of AURKA and DOCK2

Western blotting was performed to identify whether protein expression levels of AURKA and DOCK2 were reduced by the siRNA sequence selected in Example 3-1. Specifically, mesenchymal stem cells were transfected with siAURKA (Candidate 3) and siDOCK2 (Candidate 2) respectively and cultured for 72 hours, and then rinsed with PBS. Then, the cells were lysed by a RIPA buffer (BIOSESANG, Sungnam, Gyeonggi, Korea) containing a protease inhibitor cocktail (Amresco, Solon, OH, USA) and centrifuged at 4°C with 15,000 g for 30 minutes to obtain a supernatant. 30 µg of proteins were subjected to electrophoresis by SDS-PAGE for separation by size and then transferred to a polyvinylidene difluoride (PVDF) membrane. The membrane was blocked by TBST containing 5% skim milk at room temperature for 1 hour and incubated overnight with a primary antibody in the TBST containing 5% skim milk at 4°C. Then, the membrane was rinsed three times with TBST for 10 minutes and then incubated with a secondary antibody diluted in the TBST containing 5% skim milk at room temperature for 1 hour. Subsequently, the membrane was rinsed three times with TBST for 10 minutes each and treated with an ECL solution (Advansta, USA), and then images of bands were obtained using a gel imaging system (Amersham Imager 600, GE Healthcare, Buckinghamshire, UK). The expression levels of the proteins were measured using Image J software and corrected with β-actin. AURKA (Invitrogen, CA), DOCK2 (Santa Cruz Biotechnology, Dallas, TX, USA), and Beta-actin (Santa Cruz Biotechnology, Dallas, TX, USA) were used as the primary antibody.

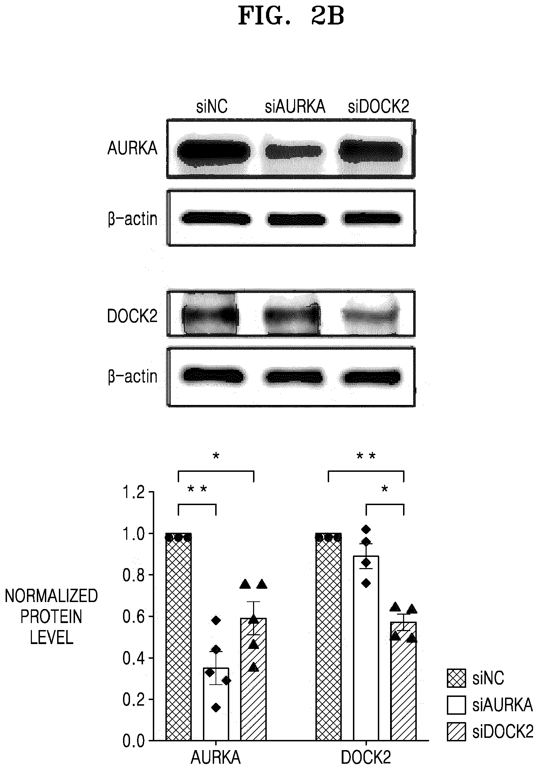

FIG. 2B shows protein expression levels of AURKA and DOCK2 after knocking down the MSMF gene by transfecting mesenchymal stem cells with siAURKA and siDOCK2.

As a result, as shown in FIG. 2B, the protein expression levels of AURKA and DOCK2 were statistically significantly decreased in the siAURKA-treated group and the siDOCK2-treated group by 0.51 ± 0.06 times and 0.76 ± 0.04 times, respectively, compared to the control group treated with siNC.

### 3-3. Identification of Sternness

Mesenchymal stem cells were knocked down by transfection with siAURKA and siDOCK2 using the sequence selected in Example 3-1 and stemness thereof was identified by FACS. Markers of the mesenchymal stem cells were identified by expression of CD44, CD73, CD90, CD105, and CD166, and hematopoietic stem cell lineage markers were compared with MSAF siNC-treated control group in stemness by using CD14, CD11b, HLA-DR (MHCII), CD34, CD45, and CD19 (BD Biosciences, USA). In this regard, 10000 events were acquired and analyzed using a BD FACS Verse flow cytometer.

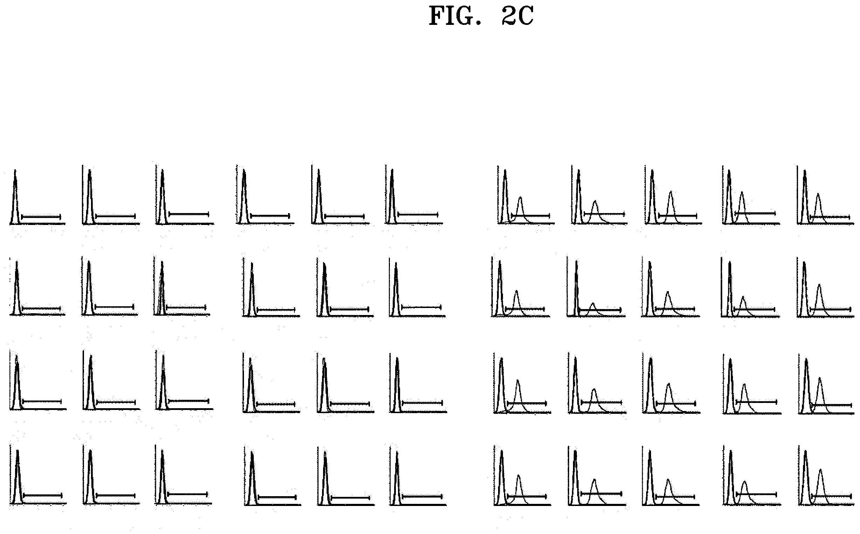

FIG. 2C is a graph showing stemness obtained by FACS after knocking down the MSMF gene by transfecting the mesenchymal stem cells with siAURKA and siDOCK2.

As a result, as shown in FIG. 2C, all of the cells expressed positive markers CD44, CD73, CD90, CD105, and CD166, but did not express negative markers CD14, CD11b, HLA-DR (MHCII), CD34, CD45, and CD19. That is, it was confirmed that stemness was not changed although AURKA and DOCK2 were knocked down by siRNA in the mesenchymal stem cells.

### Example 4. Correlation of Inhibition of AURKA and DOCK2 Expression Respectively with Migration Ability, Colony Formation Ability. Doubling Time, Cell

### Proliferation and Kinase Phosphorylation

### 4-1. Correlation between Inhibition of AURKA and DOCK2 Expression and Migration Ability

In order to identify correlation between inhibition of AURKA and DOCK2 expression and the migratory ability of mesenchymal stem cells, a wound healing assay was performed. Specifically, mesenchymal stem cells transfected with siNC, and siAURKA and siDOCK2 selected in Example 3-1 were aliquoted into a 12-well plate at a density of 1 ×10⁵ cells/well and incubated for 48 hours using a minimum essential medium (MEM) Alpha (Invitrogen-Gibco, Rockville, MD) supplemented with 10% FBS. Then, the cells were rinsed twice with the MEM Alpha not containing the FBS to inhibit proliferation of the cells and incubated in the MEM Alpha supplemented with 10 µg/ml mitomycin C (Sigma-Aldrich, St. Louis, MO) for 2 hours. Then, scratches were made using a 200 pipette tip. After rinsing 5 times with the culture solution, images were obtained at 40x magnification using a microscope after 0 and 24 hours. The obtained images were quantitatively analyzed using Image J software of Java, and cell migration ability was expressed as a percent wound closure.

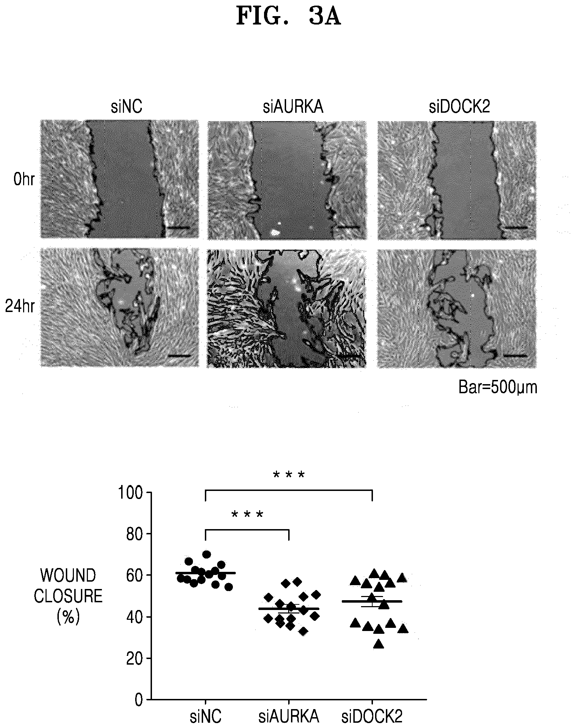

FIG. 3A shows migration ability of mesenchymal stem cells after knocking down the MSMF gene by transfecting mesenchymal stem cells with siAURKA and siDOCK2.

As a result, as shown in FIG. 3A, the migration ability of the control group treated with siNC was 61.02 ± 1.0%, whereas the migration ability of the siAURKA-treated group and the siDOCK2-treated group were 44.06 ± 1.87% and 47.18 ± 2.93%, respectively. That is, it was confirmed that the migration ability of the mesenchymal stem cells significantly decreased as the expression levels of the AURKA and DOCK2 genes decreased.

### 4-2. Correlation between Inhibition of AURKA and DOCK2 Expression and Colony Formation Ability

The correlation between inhibition of AURKA and DOCK2 expression and colony forming ability of mesenchymal stem cells was identified. Specifically, mesenchymal stem cells transfected with siNC, and siAURKA, and siDOCK2 selected in Example 3-1 were aliquoted into a 6-well plate at a density of 1 ×10³ cells/well and incubated in a MEM Alpha (Invitrogen-Gibco, Rockville, MD) supplemented with 10% FBS for 14 days. Then, the cells were rinsed with PBS and immobilized with cold 100% methanol for 20 minutes. The immobilized cells were stained with a 1% crystal violet solution (Sigma-Aldrich, St. Louis, MO) for 30 minutes and rinsed three times or more with distilled water, and only colonies consisting of more than 50 cells were counted.

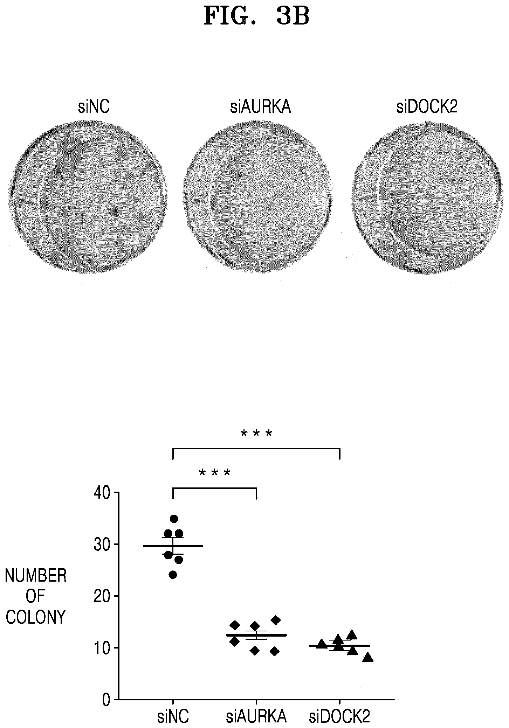

FIG. 3B is a graph showing whether mesenchymal stem cells form colonies after knocking down an MSMF gene by transfecting the mesenchymal stem cells with siAURKA and siDOCK2.

As a result, as shown in FIG. 3B, while the number of colonies was 30.7 ± 4.0 in the control group treated with siNC, the numbers of colonies were decreased to 13.0 ± 2.7 and 11.0 ± 1.4, respectively in the siRNA-treated group and the siDOCK2-treated group. That is, it was confirmed that the formation of the mesenchymal stem cells significantly decreased as the expression levels of the AURKA and DOCK2 genes decreased.

### 4-3. Correlation between Inhibition of AURKA and DOCK2 Expression and Doubling Time

The correlation between inhibition of AURKA and DOCK2 expression and doubling time of mesenchymal stem cells was identified. Specifically, mesenchymal stem cells transfected with siNC, and siAURKA, and siDOCK2 selected in Example 3-1 were aliquoted into a 12-well plate at a density of 3 ×10³ cells/cm² and incubated in a MEM Alpha (Invitrogen-Gibco, Rockville, MD) supplemented with 10% FBS. Doubling time was identified by measuring the number of cells after 0, 24, 72, and 144 hours from the aliquoting.

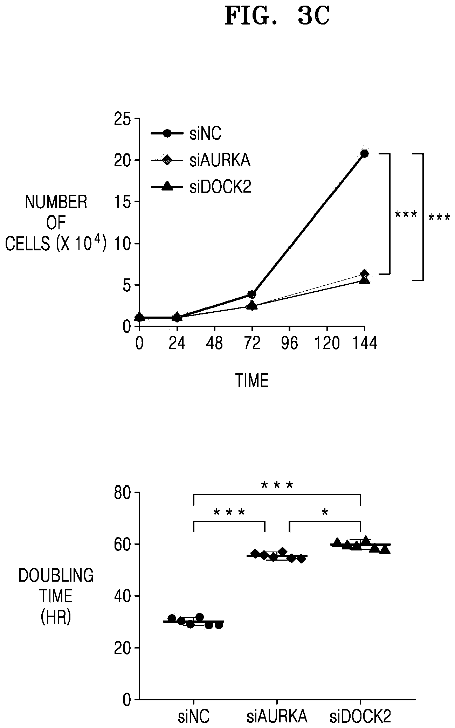

FIG. 3C is a graph showing doubling time of mesenchymal stem cells after knocking down an MSMF gene by transfecting the mesenchymal stem cells with siAURKA and siDOCK2.

As a result, as shown in FIG. 3C, while the doubling time of the control group treated with siNC was 31.1 ± 0.4 hours, the doubling times of the siAURKA-treated group and the siDOCK2-treated group were 55.3 ± 0.8 hours and 59.2 ± 0.8 hours, respectively. That is, it was confirmed that the doubling time of the mesenchymal stem cells significantly increased as the expression levels of the AURKA and DOCK2 genes decreased.

### 4-4. Correlation between Inhibition of AURKA and DOCK2 Expression and Cell Proliferative Ability

The correlation between inhibition of AURKA and DOCK2 expression and cell proliferative ability of mesenchymal stem cells was confirmed. Specifically, mesenchymal stem cells transfected with siNC, and siAURKA, and siDOCK2 selected in Example 3-1 were aliquoted into a 96-well plate at a density of 2 ×10³ cells/well and incubated in a MEM Alpha (Invitrogen-Gibco, Rockville, MD) supplemented with 10% FBS. Absorbance was measured at 450 nm using a cell counting kit-8 (CCK-8, Dojindo, Japan, Tokyo) after 0, 24, 72, and 144 hours from the aliquoting.

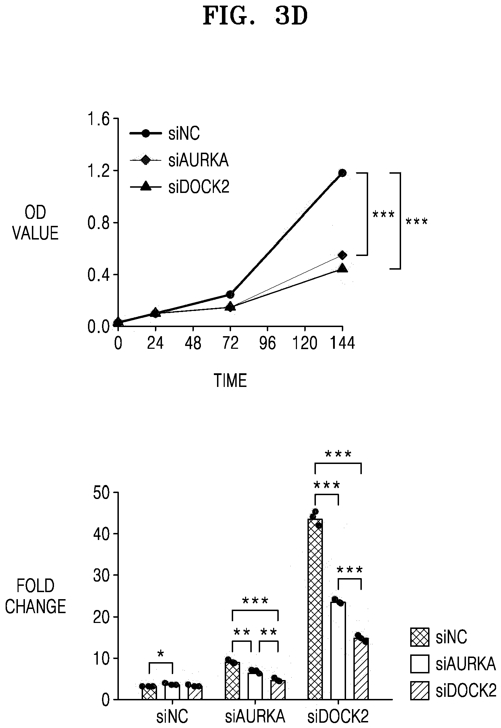

FIG. 3D shows cell proliferative ability of mesenchymal stem cells after knocking down an MSMF gene by transfecting the mesenchymal stem cells with siAURKA and siDOCK2.

As a result, as shown in FIG. 3D, there was no difference in cell proliferative ability between the siAURKA-treated and siDOCK2-treated groups and the control group treated with siNC after 24 hours of incubation of cells. However, it was confirmed that the cell proliferative ability of the siAURKA-treated and siDOCK2-treated groups decreased after 48 hours compared to the control group treated with siNC, and the decrease in the cell proliferative ability was accelerated over time.

### 4-5. Correlation between Inhibition of AURKA and DOCK2 Expression and Kinase Phosphorylation

The correlation between inhibition of AURKA and DOCK2 expression and phosphorylation of AKT, ERK, FAK and JNK was identified. Specifically, Western blotting was performed in the same manner as in Example 3-2 above, except that AKT, p-AKT, p-ERK (R&D, Minneapolis, MN, USA), ERK, FAK, p-FAK, JNK, p-JNK (Cell Signaling Technology, Danvers, MA) and Beta-actin (Santa Cruz Biotechnology, Dallas, TX, USA) were used as primary antibodies.

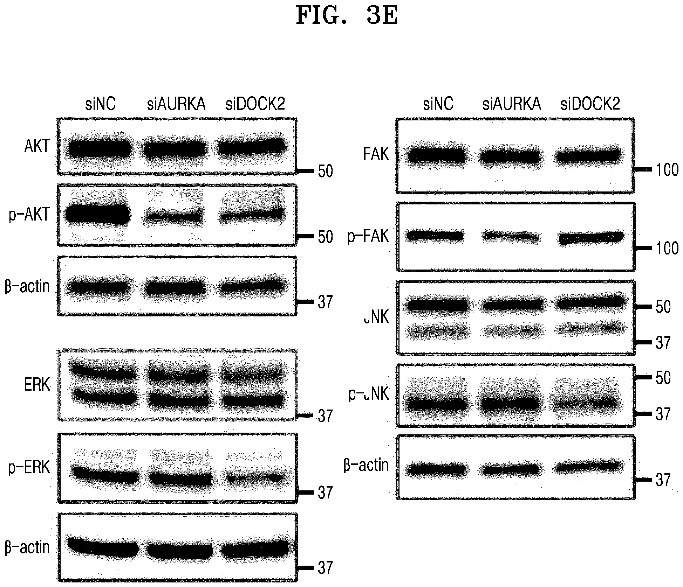

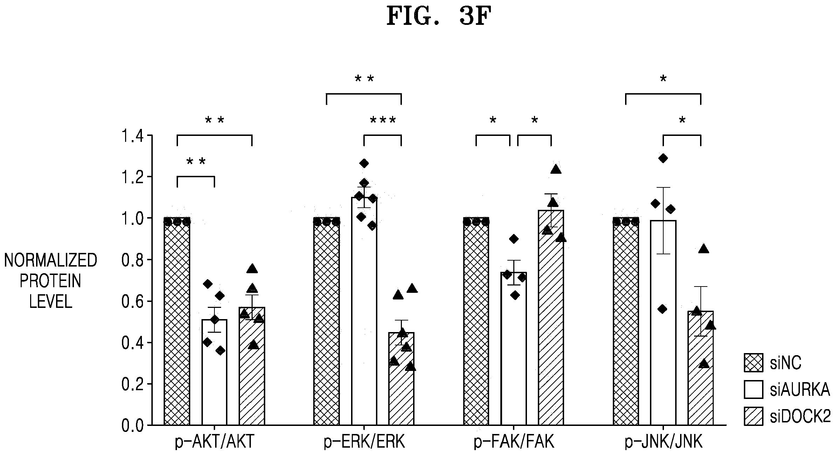

FIGS. 3E and 3F show effects of inhibition of expression of AURKA and DOCK2 genes on phosphorylation of AKT and ERK, which are kinases involved in proliferation of cells and phosphorylation of FAK and JNK, which are kinases involved in the migration ability.

As a result, as shown in FIGS. 3E and 3F, it was confirmed that the phosphorylation of AKT and FAK was inhibited by inhibiting the expression of AURKA, and the phosphorylation of AKT, ERK, and JNK was inhibited by inhibiting the expression of DOCK2. That is, it can be seen that AURKA affects cell proliferation and migration ability of cells via AKT and FAK, and DOCK2 affects cell proliferation via AKT, ERK, and JNK.

By combining the results of Example 4 above, it may be seen that the AURKA and DOCK2 genes are involved in self-maintenance by affecting the migration ability, colony formation ability, and cell proliferation of mesenchymal stem cells.

### Example 5. Correlation of Expression Level of AURKA and DOCK2 respectively with Doubling Time and Kinase Phosphorylation

### 5-1. Correlation between mRNA Expression Level of AURKA and DOCK2 of Mesenchymal Stem Cell and Doubling Time

Due to a problem of large donor variation of mesenchymal stem cells, attempts have been made to select mesenchymal stem cells having reduced donor variation by identifying correlation between mRNA expression levels of AURKA and DOCK2 of the mesenchymal stem cells and doubling time. Specifically, 10 mesenchymal stem cells obtained from 10 different donors were aliquoted into a 25T flask at a density of 3 ×10³ cells/cm² and incubated in a MEM Alpha (Invitrogen-Gibco, Rockville, MD) supplemented with 10% FBS. When the cells grew by about 80%, RNA was extracted and qRT-PCR was performed, and then relative mRNA expression levels of AURKA and DOCK2 were compared. In addition, after identifying doubling time of the 10 mesenchymal stem cells, the correlation between the expression levels of AURKA and DOCK2 genes and the doubling time of cells was identified by Pearson correlation analysis.

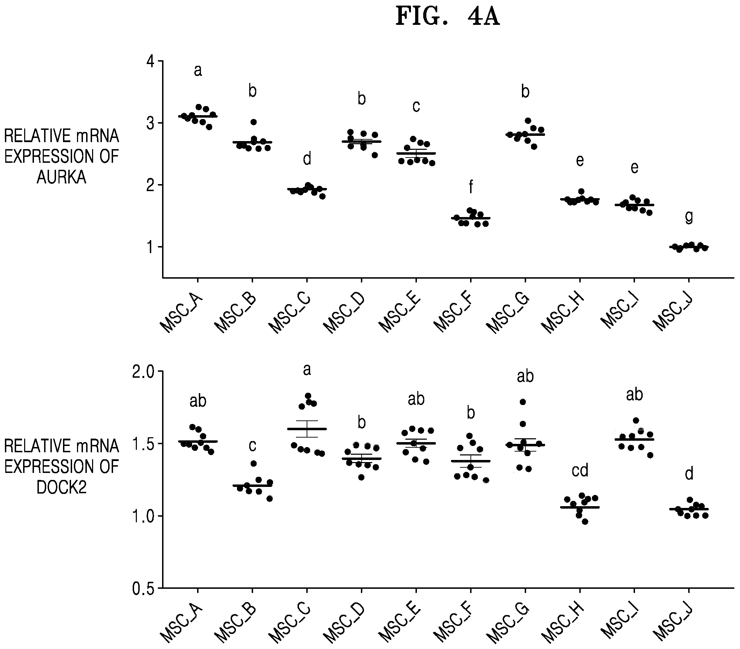

FIG 4A is a graph showing comparison of relative mRNA expression levels between AURKA and DOCK2 in 10 different mesenchymal stem cells.

As a result, as shown in FIG. 4A, MSC_A had the highest mRNA expression level of AURKA among the 10 stem cells, and MSC_C had the highest mRNA expression level of DOCK among the 10 stem cells. On the other hand, MSC_J had the lowest mRNA expression levels of AURKA and DOCK2.

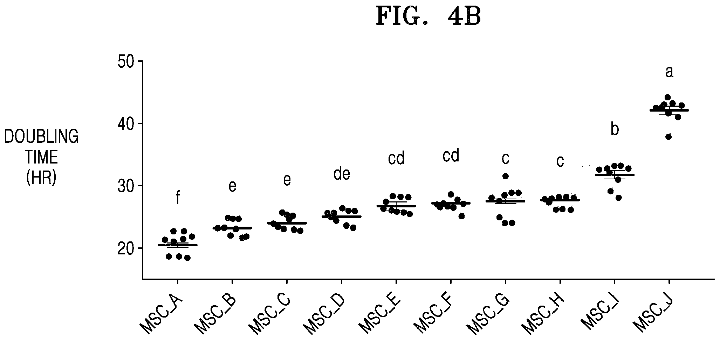

FIG. 4B is a graph sequentially showing doubling time of 10 different mesenchymal stem cells.

As a result, as shown in FIG. 4B, the doubling time of MSC_A was the shortest as 20.9 ± 0.6 hours, and the doubling time of MSC_J was the longest as 41.9 ± 0.6 hours. An average doubling time of the 10 mesenchymal stem cells was 27.6 ± 1.8 hours.

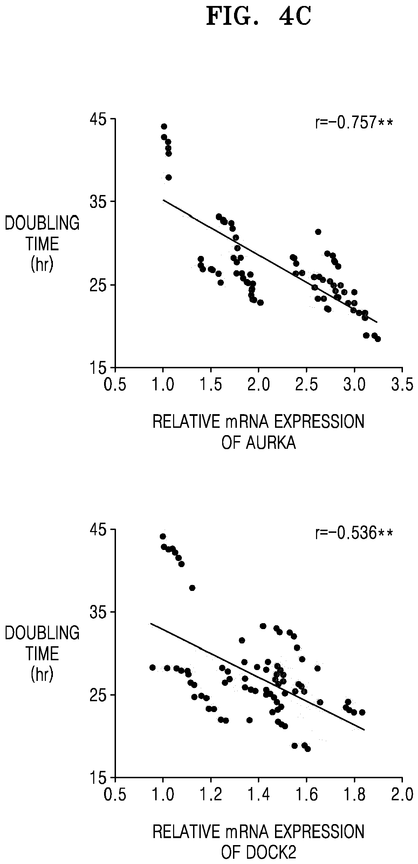

FIG. 4C shows graphs of correlation between the mRNA expression levels of AURKA and DOCK2 and doubling time.

As a result, as shown in FIG. 4C, the correlation between the mRNA expression level of AURKA and the doubling time was a very strong negative correlation as r = - 0.757, and the correlation between the mRNA expression level of DOCK2 and the doubling time was a strong negative correlation as r = -0.536 (p < 0.01).

Thus, as the expression levels of AURKA and DOCK2 genes increase in mesenchymal stem cells, the doubling time decreases. Therefore, mesenchymal stem cells having high self-proliferative ability may be easily selected by measuring the expression levels of AURKA and DOCK2 genes.

### 5-2. Correlation between mRNA Expression Level of AURKA and DOCK2 and Protein Expression Level of AURKA and DOCK2 in Mesenchymal Stem Cell

The correlation between mRNA expression levels of AURKA and DOCK2 and protein expression levels of AURKA and DOCK2 was identified in mesenchymal stem cells. Specifically, Western blotting was performed in the same manner as in Example 3-2, except that mesenchymal stem cells cultured in a growth medium by about 80% were used. Subsequently, MSC_A having the highest mRNA expression level of AURKA, MSC_C having the highest mRNA expression level of DOCK2, and MSC_J having relatively low mRNA expression levels of AURKA and DOCK2 were selected (see FIG. 4A), and a difference in protein expression levels of AURKA and DOCK2 was identified.

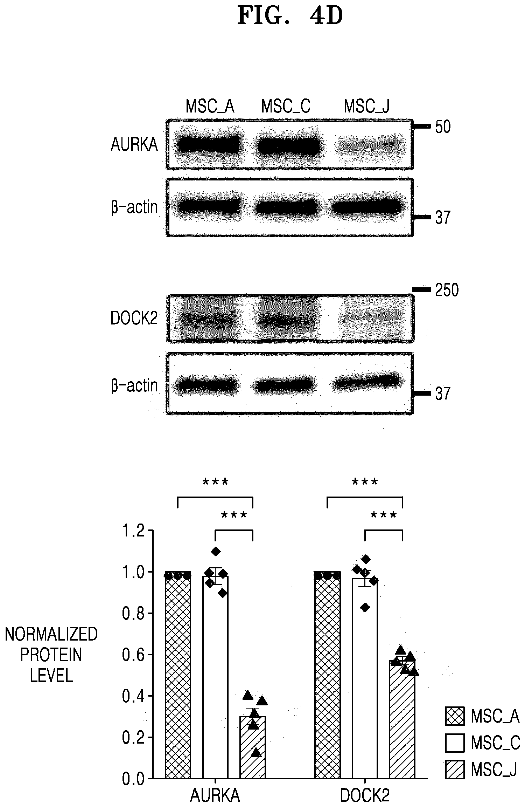

FIG. 4D shows protein expression levels of AURKA and DOCK2 in mesenchymal stem cells.

As shown in FIG. 4D, the protein expression levels of AURKA and DOCK2 in MSC_J decreased by 0.31 ± 0.05 times and 0.59 ± 0.02 times, respectively, compared to those of MSC_A and MSC_C. That is, it was confirmed that in the mesenchymal stem cells having low mRNA expression levels of AURKA and DOCK2, the expression levels of the proteins exhibiting the functions thereof also decreased.

### 5-3. Correlation between mRNA Expression Level of AURKA and DOCK2 and Kinase Phosphorylation in Mesenchymal Stem Cell

Western blotting was performed in the same manner as in Examples 4-5 to identify the correlation between mRNA expression levels of AURKA and DOCK2 and phosphorylation of AKT, ERK, FAK, and JNK in mesenchymal stem cells.

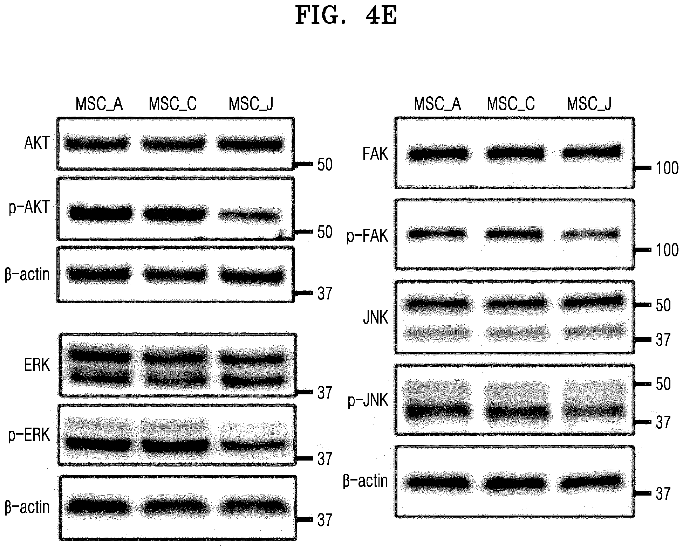

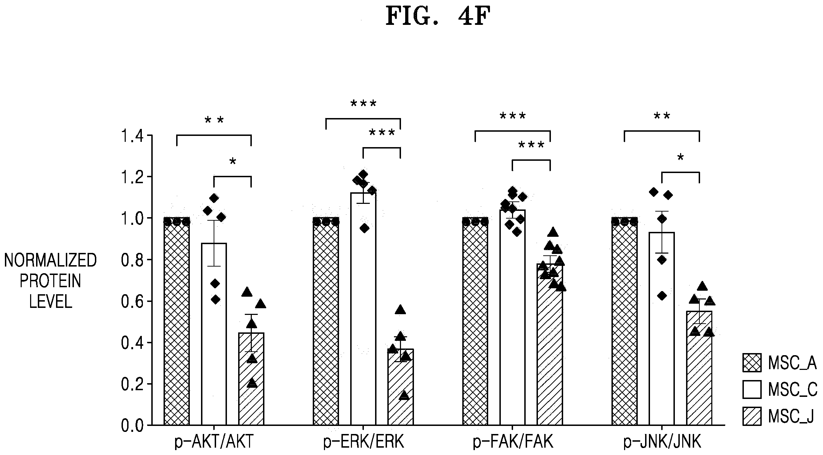

FIGS. 4E and 4F show results of confirming phosphorylation levels of AKT, ERK, FAK, and JNK in mesenchymal stem cells.

As a result, as shown in FIGS. 4E and 4F, in the case of MSC_A and MSC_C, there was no significant difference in the phosphorylation of AKT and ERK, which are kinases involved in cell proliferation, and phosphorylation of FAK and JNK, which are kinases involved in migration ability. However, in the case of MSC_J, it was confirmed that phosphorylation of AKT was reduced by 0.46 ± 0.08 times and phosphorylation of ERK was reduced by 0.37 ± 0.07 times compared to those of MSC_A and MSC_C. In addition, it was confirmed that phosphorylation of FAK was reduced by 0.79 ± 0.03 times and phosphorylation of JNK was reduced by 0.56 ± 0.05 times.

Therefore, the difference of mRNA expression levels of AURKA and DOCK2 and protein expression levels of AURKA and DOCK2 affects phosphorylation of kinases involved in cell proliferation and migration ability in mesenchymal stem cells, and mesenchymal stem cells having high expression levels of these genes may promote cell proliferation via phosphorylation of AKT and/or ERK and promote the migration ability via phosphorylation of FAK and/or JNK.

### Example 6. Correlation between Expression Level of AURKA and DOCK2 of Mesenchymal Stem Cell and Apoptosis

### 6-1. Identification of Expression of Apoptosis-related Protein According to Gene Expression Level of AURKA and DOCK2 of Mesenchymal Stem Cell

In order to identify an inhibitory effect of mesenchymal stem cells on apoptosis, the correlation between expression levels of AURKA and DOCK2 and expression of cleaved PARP and cleaved Caspase 3 was evaluated. Specifically, muscle cells C2C12 were aliquoted into a 6-well plate at a density of 8 ×10³ cells/cm² and incubated in a Dulbecco's Modified Eagle's Medium (DMEM, Biowest SAS, Nuaille, France) supplemented with 10% FBS and 1 U/ml penicillin/streptomycin (Gibco BRL, Grand Island, NY). After 24 hours of incubation, apoptosis was induced by starvation of FBS for another 24 hours. Then, the muscle cells in which apoptosis was induced were co-cultured with mesenchymal stem cells by insertion. Western blotting was performed using cleaved Poly ADP ribose polymerase PARP (Cell Signaling Technology, Danvers, MA), cleaved Caspase 3 (Cell Signaling Technology, Danvers, MA), and Beta-actin (Santa Cruz Biotechnology, USA) as primary antibodies and the degree of apoptosis was compared.

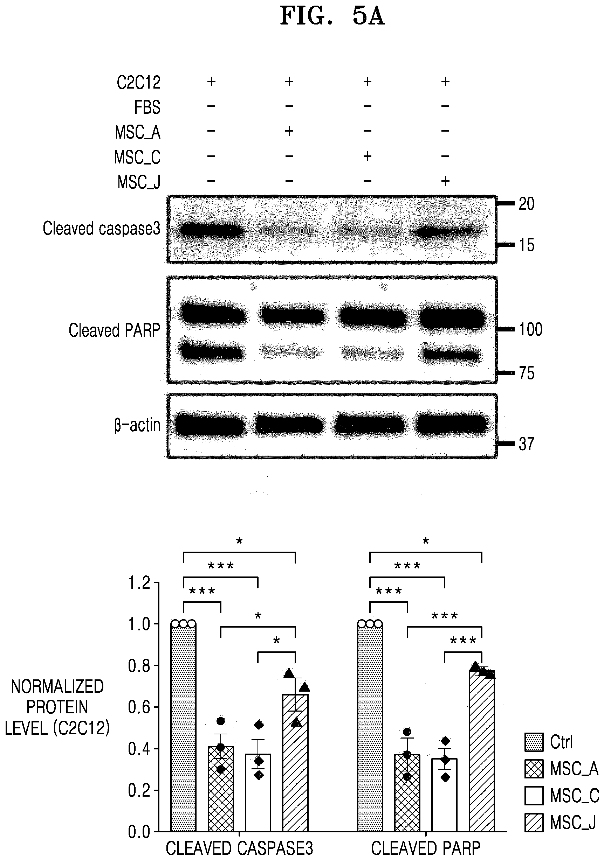

FIG. 5A shows results of identifying expression of apoptosis-related proteins according to mRNA expression levels of AURKA and DOCK2, by co-culturing mesenchymal stem cells and muscle cells in which apoptosis is induced.

As a result, as shown in FIG. 5A, it was confirmed that the expression levels of the cleaved PARP and the cleaved Caspase 3 decreased in an experimental group co-cultured with the mesenchymal stem cells compared to the control group in which apoptosis was induced. Specifically, compared to the control group in which apoptosis was induced, the expression levels of the cleaved caspase3 decreased by 0.41 ± 0.07 and 0.38 ± 0.07 times, respectively, and the expression levels of the cleaved PARP significantly decreased by 0.37 ± 0.06 and 0.35 ± 0.05 times, respectively, in the experimental groups co-cultured with MSC_A or MSC_C (p<0.001). However, in the experimental group co-cultured with MSC_J, the expression level of the cleaved caspase3 was 0.66 ± 0.07 times, and the expression level of the cleaved PARP was 0.78 ± 0.01 times indicating slight differences from the control group in which apoptosis was induced. That is, it may be seen that the ability to inhibit apoptosis of muscle cells deteriorated as the mRNA expression levels of AURKA and DOCK2 in mesenchymal stem cells decrease.

### 6-2. Identification of Degree of Reduction in Apoptosis According to Gene Expression Level of AURKA and DOCK2 of Mesenchymal Stem Cell

Live/dead staining was performed using an Apoptosis/Necrosis Detection Kit (Abcam, Cambridge, MA, USA) to identify the degree of reduction in apoptosis of C2C12 cells of Example 6-1. Specifically, the experimental group, in which the mesenchymal stem cells and the apoptosis-induced muscle cells were co-cultured in the same manner as in Example 6-1, was rinsed twice with PBS, followed by reaction with an Apopxin Green Indicator capable of detecting apoptosis and a CytoCalcein Violet 450 capable of detecting live cells in a light-blocked condition for 1 hour. Then, the cells were rinsed twice with PBS and images were obtained by using a fluorescence microscope. Among total cells, ratios of apoptotic cells were measured using Image J.

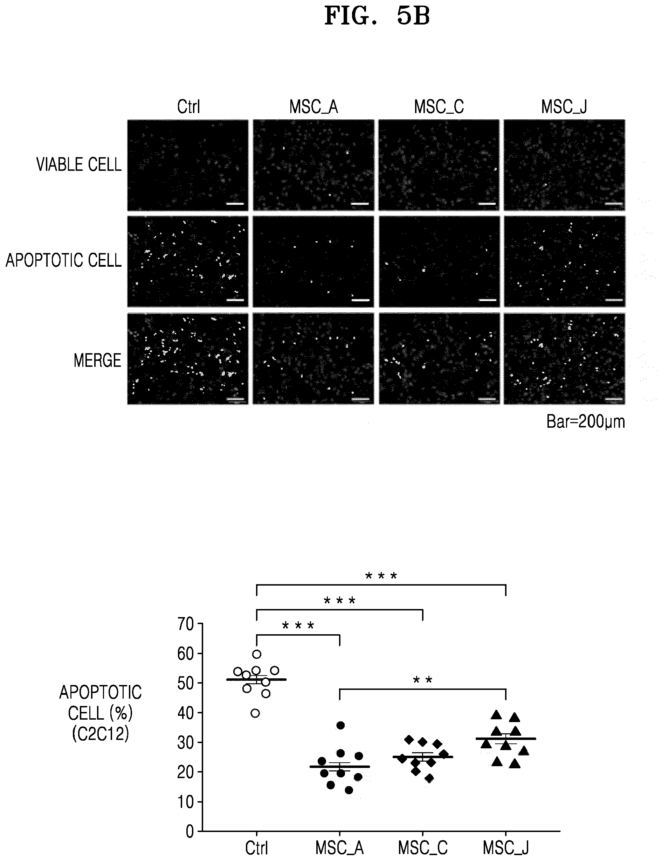

FIG. 5B shows results of identifying degrees of reduction in apoptosis according to mRNA expression levels of AURKA and DOCK2, by co-culturing mesenchymal stem cells and muscle cells in which apoptosis is induced.

As a result, as shown in FIG. 5B, apoptosis was inhibited in the experimental group co-cultured with the mesenchymal stem cells compared to the control group in which apoptosis was induced. Specifically, while the percentage of apoptotic cells was 52.04 ± 1.93% in the control group in which apoptosis was induced, the percentages of apoptotic cells decreased to 22.50 ± 2.17%, 25.72 ± 1.53%, and 31.87 ± 1.99% in the experimental groups co-cultured with MSC_A, MSC_C, or MSC_J (p<0.001). In particular, in the case of the experimental group co-cultured with MSC_A having the highest mRNA expression level of AURKA, the ratio of apoptotic cells was significantly reduced compared to the experimental group co-cultured with MSC_J (p<0.001).

Therefore, it may be seen that the mesenchymal stem cells having low mRNA expression levels of AURKA and DOCK2 have decreased inhibitory effects on apoptosis when co-cultured with the apoptosis-induced muscle cells.

### Example 7. Identification of Correlation between Inhibition of Expression of AURKA and DOCK2 Genes and Apoptosis

### 7-1. Identification of Expression of Apoptosis-related Protein According to Inhibition of Expression of AURKA and DOCK2 Genes

In order to identify whether the knock-down of the AURKA and DOCK2 genes affects the apoptosis inhibitory effect, apoptosis-induced muscle cells were co-cultured respectively with mesenchymal stem cells and mesenchymal stem cells treated with siNC, and siAURKA or siDOCK2 selected in Example 3-1, and then protein expression was identified in the same manner as in Example 6-1.

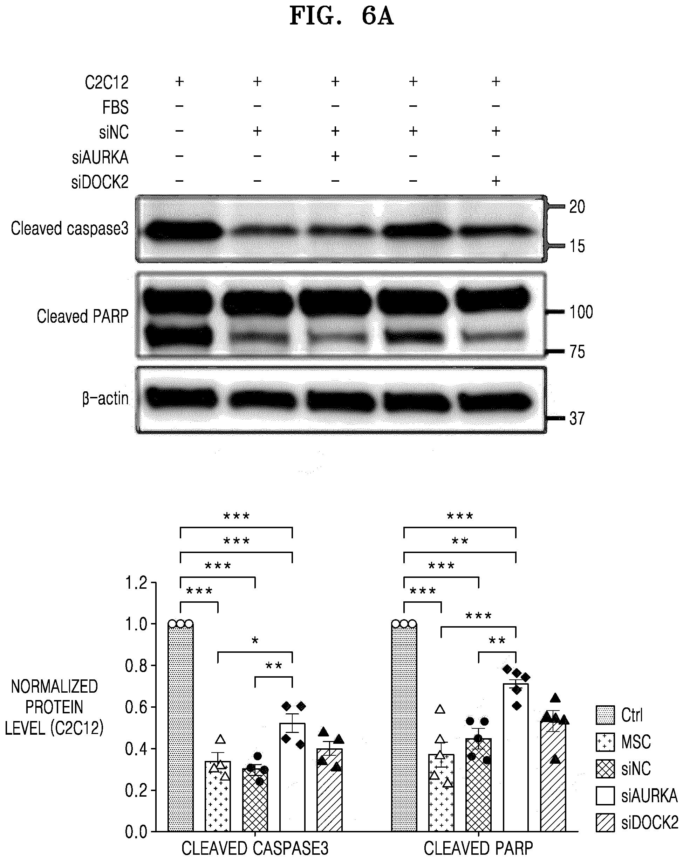

FIG. 6A shows results of confirming expression of apoptosis-related proteins according to inhibition of expression of AURKA and DOCK2 genes, by co-culturing mesenchymal stem cells in which AURKA and DOCK2 genes are knocked down and muscle cells in which apoptosis is induced.

As a result, as shown in FIG. 6A, protein expression of the cleaved caspase3 and the cleaved PARP was significantly decreased in an experimental group co-cultured with the mesenchymal stem cells compared to a control group (apoptosis-induced muscle cells). Specifically, it was confirmed that the expression levels of the cleaved caspase3 and the cleaved PARP were decreased by 0.34±0.04, 0.30±0.02, and 0.52 ± 0.05 and 0.40 ± 0.04 times, respectively in the muscle cells co-cultured with the mesenchymal stem cells, the siNC-treated group, the siAURKA-treated group, and the siDOCK2-treated group compared to the control group in which apoptosis was induced (p<0.001). In addition, it was confirmed that the expression levels of the cleaved PARP were decreased by 0.39 ± 0.07, 0.45 ± 0.04, and 0.72 ± 0.043, and 0.54 ± 0.045 times, respectively in the muscle cells co-cultured with the mesenchymal stem cells, the siNC-treated group, the siAURKA-treated group, and the siDOCK2-treated group compared to the control group in which apoptosis was induced (MSC, siNC, and siDOCK2, p<0.001; and siAURKA, p<0.01). In particular, it was confirmed that the expression levels of the cleaved caspase3 and the cleaved PARP were statistically significantly increased in the siAURKA-treated group co-cultured with the apoptosis-induced muscle cells compared to the siNC-treated group (p<0.01).

### 7-2. Identification of Degree of Reduction in Apoptosis by Inhibiting Expression of AURKA and DOCK2 Genes

In order to identify the degree of reduction in apoptosis according to inhibition of expression of AURKA and DOCK2 genes, the apoptosis-induced muscle cells were co-cultured respectively with mesenchymal stem cells, and siNC, and siAURKA or siDOCK2 selected in Example 3-1 above, followed by evaluation in the same manner as in Example 6-2.

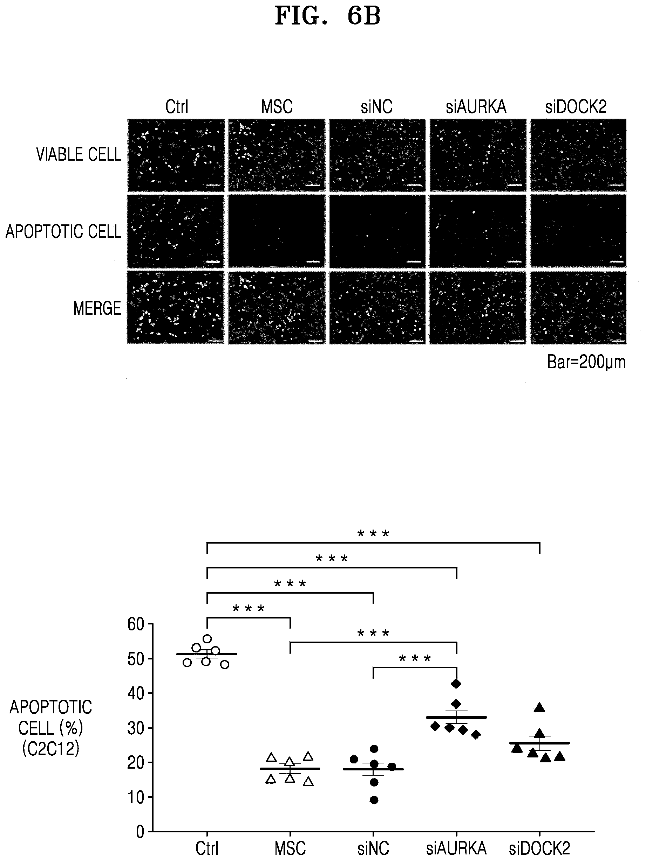

FIG. 6B shows results of identifying degrees of reduction in apoptosis according to inhibition of expression of AURKA and DOCK2 genes, by co-culturing mesenchymal stem cells in which AURKA and DOCK2 genes are knocked down and muscle cells in which apoptosis is induced.

As a result, as shown in FIG. 6B, it was confirmed that apoptosis was significantly inhibited in the experimental group co-cultured with the mesenchymal stem cells compared to the control group (apoptosis-induced muscle cells). Specifically, while an apoptosis rate was 41.73 ± 1.03% in the control group in which apoptosis was induced, the apoptosis rates were decreased to 17.93 ± 1.40%, 17.79 ± 2.06%, 32.75 ± 2.24%, and 25.62 ± 2.29%, respectively, in the case of the muscle cells co-cultured with the mesenchymal stem cells, the siNC-treated group, the siAURKA-treated group, and the siDOCK2-treated group (p<0.001). Particularly, it was confirmed that the apoptosis rates significantly increased in the siAURKA-treated group co-cultured with apoptosis-induced muscle cells compared to the siNC-treated group (p<0.001).

Therefore, because the inhibitory effect on apoptosis more significantly decreases by knocking down AURKA, it is considered that the inhibitory effect of the mesenchymal stem cells on apoptosis of muscle cells is more affected by the AURKA than by the DOCK2.

### 7-3. Identification of Change in Protein Expression Level of XCL1 According to Inhibition of Expression of AURKA Genes

It was identified whether the inhibitory effect of the mesenchymal stem cells on apoptosis of muscle cells is directly related to the expression level of AURKA gene. Specifically, after knocking down the AURKA gene in mesenchymal stem cells using the siRNA selected in Example 3-1, the inhibitory effect on apoptosis was evaluated. Western blotting was performed in the same manner as in Example 3-2, except that XCL1 (R&D, Minneapolis, MN, USA) and Beta-actin (Santa Cruz Biotechnology, USA) were used as primary antibodies.

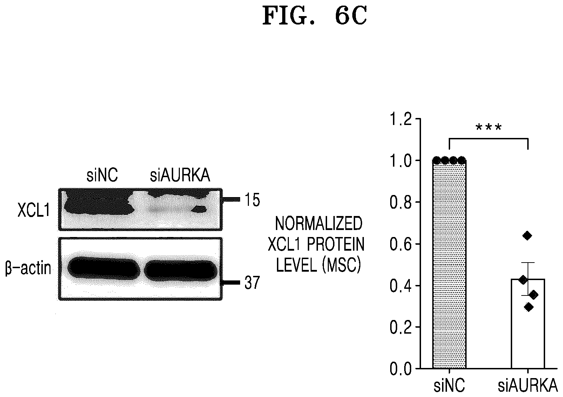

FIG. 6C shows protein expression levels of XCL1 of mesenchymal stem cells in which an AURKA gene is knocked down.

As a result, as shown in FIG. 6C, as expression of AUKRA gene was inhibited, the expression level of XCL1 that is a protein involved in inhibition of apoptosis was decreased. Specifically, the expression level of XCL1 of the siAURKA-treated group decreased by 0.44 ± 0.07 times compared to that of the siNC-treated group (p<0.01). That is, the AURKA gene may exhibit the inhibitory effect of the mesenchymal stem cells on apoptosis of muscle cells by directly affecting expression of XCL1.

### Example 8. Identification of Correlation between mRNA Expression Level of AURKA of Mesenchymal Stem Cell and Apoptosis of Muscle Tissue

### 8-1. Identification of Inhibition of Apoptosis of Muscle Cell according to mRNA Expression Level of AURKA of Mesenchymal Stem Cell

An inhibitory effect of mesenchymal stem cells on apoptosis of muscle cells according to a mRNA expression level of AURKA was identified by Annexin V staining. Specifically, mesenchymal stem cells were administered to the tail vein of a muscular dystrophy mouse model (Mdx) once at dose of 1 × 10⁵ cells/100 µl. In this regard, based on the results of Example 7 above, MCS_A having the highest mRNA expression level of AURKA, MCS_E havin a median mRNA expression level of AURKA, and MCS_J having the lowest mRNA expression level of AURKA which are determined to directly affecting inhibition of apoptosis were used. After one week, the mice were sacrificed and tissues of calf muscles were separated. Then, expression levels of proteins were identified in the same manner as in Example 3-2 except that annexin V (Abcam, Cambridge, Ma, USA) and Beta-actin (Santa Cruz Biotechnology, Dallas, TX, USA) were used as primary antibodies.

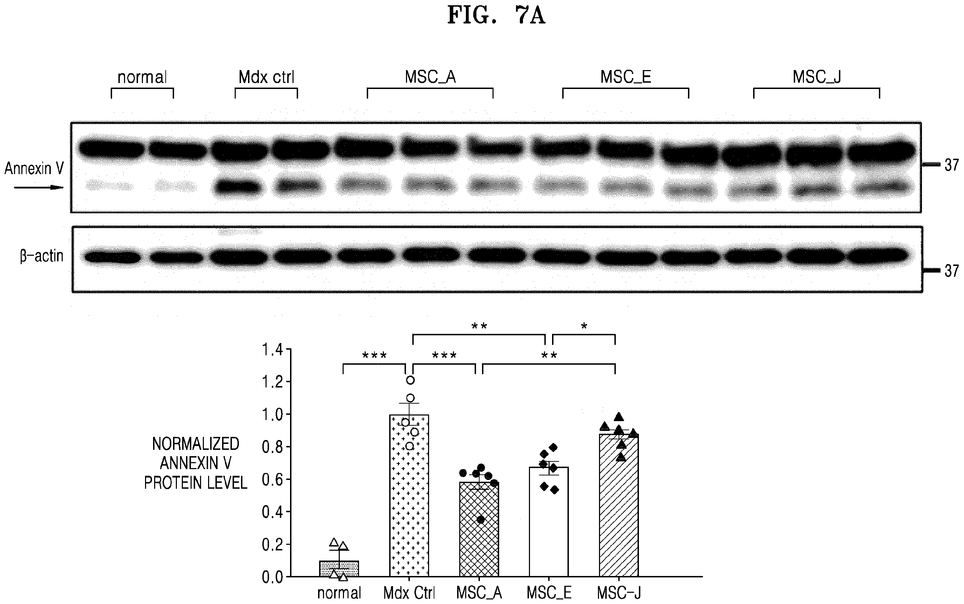

FIG. 7A shows inhibitory effects on apoptosis of muscle tissue according to a difference in mRNA expression levels of AURKA of mesenchymal stem cells in a muscular dystrophy mouse model.

As a result, as shown in FIG. 7A, although the expression levels of Annexin V decreased by 0.58 ± 0.05 and 0.67 ± 0.04 times, respectively, in the case of administering MCS_A and MCS_E, compared to the Mdx control group, there was no statistically significant difference between the MCS_J-administered group and the control group. That is, it was confirmed that the inhibitory effect on apoptosis decreased as the mRNA expression level of AURKA decreased.

### 8-2. Identification of Degree of Apoptosis of Muscle cell According to mRNA Expression level of AURKA of Mesenchymal Stem Cell

The degree of inhibition of apoptosis of muscle tissues according to the mRNA expression level of AURKA was identified by Annexin V staining. Specifically, after separating the thigh and calf muscles of the Mdx mouse model in the same manner as in Example 8-1, tissue cut by paraffin sectioning was rinsed and reacted at room temperature using a 5% blocking solution. Subsequently, the resultant was reacted with Annexin antibody (Abcam, Cambridge, UK) diluted to a 1/500 concentration at 4°C for 18 hours. After rinsing the resultant tissue, the tissue was reacted with Alexa Fluor^{®} 594 AffiniPure Goat Anti-rabbit IgG (H+L) secondary antibody (Thermo fisher scientific, Rockford, IL) at room temperature for 1 hour, followed by counter-staining using Hoechst 33342 (Thermo fisher scientific, Rockford, IL), and then fluorescence images were obtained from the tissue using a Carl Zeiss LSM 700 confocal microscopy system. The obtained images were quantitatively analyzed using Image J software of Java, and relative values to the Mdx control group were obtained.

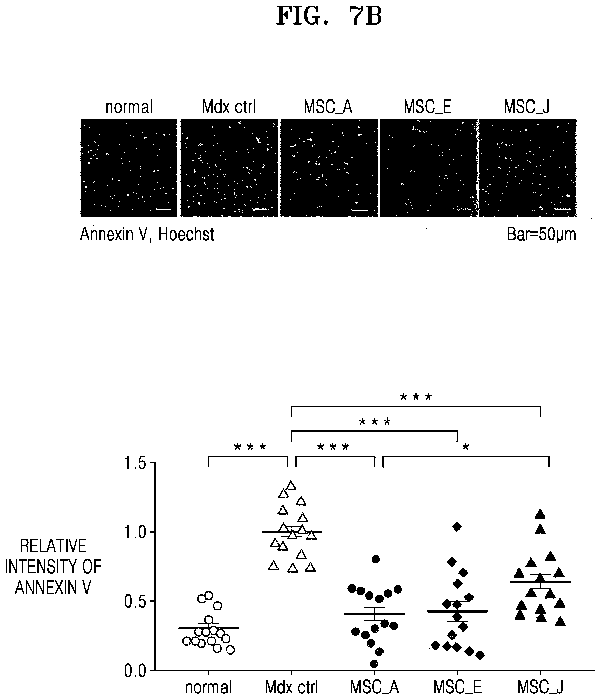

FIG. 7B shows differences in inhibitory effects on apoptosis of muscle tissue according to differences in mRNA expression levels of AURKA of mesenchymal stem cells in a muscular dystrophy mouse model.

As a result, as shown in FIG. 7B, in the case of administering MCS_A, MCS_F, and MCS_L, relative intensity of Annexin V decreased by 0.41 ± 0.05, 0.43 ± 0.07, and 0.64 ± 0.06 times compared to the Mdx control group. Particularly, in the case of administering MCS_A having the highest expression of AURKA, the relative intensity of Annexin V was significantly decreased compared to the case of administering MCS_J having the lowest expression level of AURKA. That is, the mesenchymal stem cells having a higher mRNA expression level of AURKA have a greater inhibitory effect on apoptosis of damaged muscle cells, and therefore, the mesenchymal stem cells may be used as a therapeutic agent for muscle diseases.

### Example 9. Identification of mRNA Expression Level of AURKA of Mesenchymal Stem Cell and Inhibitory Effect on Fibrosis of Muscle Tissue

### 9-1. Identification of Residual Degree of Muscle Tissue According to mRNA Expression Level of AURKA of Mesenchymal Stem Cell

An inhibitory effect of mesenchymal stem cells remaining on fibrosis of muscle tissue according to the number of mesenchymal stem cells remaining in leg muscles of a muscular dystrophy mouse model (Mdx) was identified. Specifically, after separating the thigh and calf muscles of the Mdx mouse model in the same manner as in Example 8-1, DNA was extracted therefrom using a Gentra Puregene Tissue kit (Qiagen Inc.), andreal-time PCR was performed using human Alu primers.

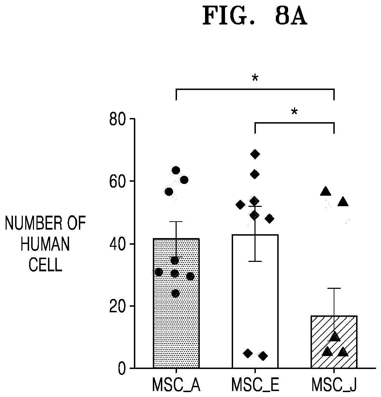

FIG. 8A is a graph showing the degree of cell survival according to mRNA expression levels of AURKA of mesenchymal stem cells in a muscular dystrophy mouse model.

As a result, as shown in FIG. 8A, the numbers of mesenchymal stem cells remaining in the leg muscles of the muscular dystrophy mouse model administered with MSC_A, MSC_F and MSC_J were 41.23 ± 5.60, 42.68 ± 8.71, and 16.96 ± 8.44, respectively. Particularly, it was confirmed that the number of the mesenchymal stem cells remaining in the leg muscles of the muscular dystrophy mouse model was the lowest in the case of administering MSC_J having the lowest mRNA expression level of AURKA. That is, the mesenchymal stem cells having a higher mRNA expression level of AURKA migrate to damaged muscle cells and a larger number of cells remain in the damaged area, thereby assisting recovery of muscles.

### 9-2. Identification of Inhibition of Fibrosis of Muscle cell According to mRNA Expression Level of AURKA of Mesenchymal Stem Cell

The inhibitory effect on fibrosis of muscle tissue according to the mRNA expression level of AURKA was identified by using expression of fibronectin. Specifically, expression of proteins was identified in the same manner as in Example 8-2, except that Fibronectin (Abcam, Cambridge, Ma, USA) and Beta-actin (Santa Cruz Biotechnology, Dallas, TX, USA) were used as primary antibodies.

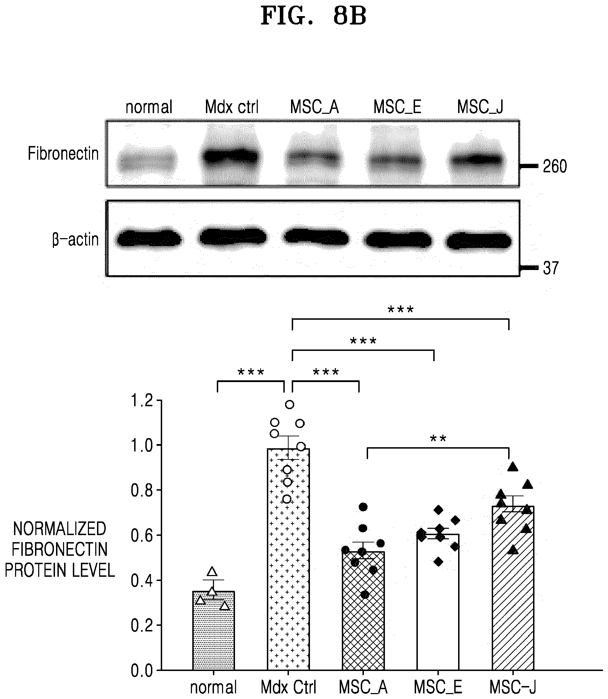

FIG. 8B shows inhibitory effects on fibrosis of muscle tissue according to mRNA expression levels of AURKA of mesenchymal stem cells in a muscular dystrophy mouse model.

As a result, as shown in FIG. 8B, the expression level of fibronectin was decreased by 0.54 ± 0.04, 0.60 ± 0.03, and 0.71 ± 0.05 times, respectively, in the case of administering MCS_A, MCS_E, and MCS_J, compared to the control group. That is, mesenchymal stem cells having a high mRNA expression level of AURKA may effectively inhibit fibrosis of damaged muscle cells by reducing expression of fibronectin.

### 9-3. Identification of Fibrosis of Muscle Cell According to mRNA Expression Level of AURKA of Mesenchymal Stem Cell

The inhibitory effect on fibrosis of muscle tissue according to the mRNA expression level of AURKA was identified by accumulation of collagen. Specifically, after rinsing the thigh muscle tissue of the Mdx mouse model in the same manner as in Example 8-1, reaction was conducted at room temperature for 1 hour using a picro-sirius red (Solution A). Then, the resultant was rinsed twice with acidified water (Solution B) and mounted, and then images thereof were obtained using a Scanscope. The obtained images were quantitatively analyzed using Image J software of Java to obtain relative values to the Mdx control group.

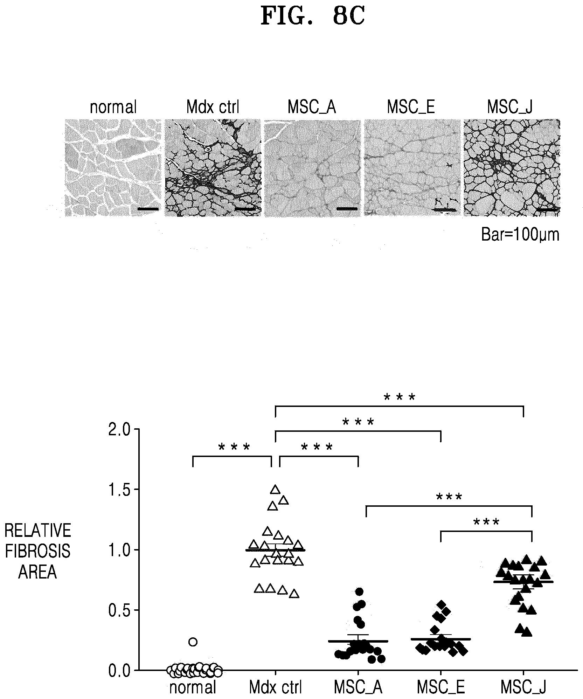

FIG. 8C shows degrees of fibrosis of muscle tissue according to mRNA expression level of AURKA of mesenchymal stem cells in a muscular dystrophy mouse model.

As a result, as shown in FIG. 8C, collagen accumulation was reduced by 0.26 ± 0.04, 0.27 ± 0.03, and 0.73 ± 0.04 times, respectively, in the case of administering MCS_A, MCS_E, and MCS_J compared to the control group. That is, mesenchymal stem cells having a high mRNA expression level of AURKA may effectively inhibit fibrosis of damaged muscle cells by reducing collagen accumulation.

The above description of the present disclosure is provided for the purpose of illustration, and it would be understood by those skilled in the art that various changes and modifications may be made without changing technical conception and essential features of the present disclosure. Thus, it is clear that the above-described embodiments of the present disclosure are illustrative in all aspects and do not limit the present disclosure.

## Claims

1. A method for selecting mesenchymal stem cells having improved self-maintenance ability, the method comprising measuring an expression or activity level of a mesenchymal stem cell (MSC) self-maintenance factor (MSMF) after culturing mesenchymal stem cells.

2. The method according to claim 1, wherein the mesenchymal stem cells are derived from umbilical cord, umbilical cord blood, bone marrow, fat, muscle nerve, skin, amnion, chorion, decidua, or placenta.

3. The method according to claim 1, wherein the MSMF is a gene involved in adhesion, differentiation, chemotaxis, or proliferation.

4. The method according to claim 3, wherein the gene is selected from FBKN4, OLR1, TNFAIP6, ANXA3, ILO6, POU2F2, TNFAIP2, SERPINE2, INHBA, VEGRA, HMGB1, CSF2, GATA3, PCSK6, SYN1, F2RL1, DOCK2, SLCOQ4, STX1B, RARRES2, CXCL1, FGF7, PLAU, SCG2, NR4A3, CORD01A, CHRM3, NPR3, BST2M, GATA4M, CREG1M, FGF7M, TPEL5, and AURKA.

5. Mesenchymal stem cells selected by the method according to claim 1.

6. The mesenchymal stem cells according to claim 5, wherein the mesenchymal stem cells have increased expression of MSMF.

7. Mesenchymal stem cells genetically engineered to secrete a mesenchymal stem cell (MSC) self-maintenance factor (MSMF) or to overexpress MSMF compared to parental cells.

8. A pharmaceutical composition for preventing or treating muscle diseases, comprising mesenchymal stem cells genetically engineered to secrete a mesenchymal stem cell (MSC) self-maintenance factor (MSMF) or to overexpress MSMF compared to parent cells, or comprising MSMF.

9. A pharmaceutical composition for preventing or treating muscle diseases, comprising a mesenchymal stem cell (MSC) self-maintenance factor (MSMF) as an active ingredient.

10. The pharmaceutical composition according to claim 9, further comprising mesenchymal stem cells.

11. The pharmaceutical composition according to claim 9, wherein the MSMF is isolated from mesenchymal stem cells.

12. A health functional food composition for preventing or alleviating muscle diseases, comprising mesenchymal stem cells genetically engineered to secrete a mesenchymal stem cell (MSC) self-maintenance factor (MSMF) or overexpress MSMF compared to parent cells, or comprising MSMF.

13. A cell therapy product comprising, as an active ingredient, mesenchymal stem cells genetically engineered to secrete a mesenchymal stem cell (MSC) self-maintenance factor (MSMF) or to overexpress MSMF compared to parent cells.

14. The cell therapy product according to claim 13, for treating muscle diseases, inflammatory diseases, immune diseases, or cancer.

15. A method for preventing or treating muscle diseases, the method comprising administering mesenchymal stem cells genetically engineered to secrete a mesenchymal stem cell (MSC) self-maintenance factor (MSMF) or overexpress MSMF compared to parent cells, or a pharmaceutically effective dose of MSMF, to an individual in need thereof.

16. A use of a composition comprising mesenchymal stem cells genetically engineered to secrete a mesenchymal stem cell (MSC) self-maintenance factor (MSMF) or overexpress MSMF compared to parent cells, or comprising MSMF, for treating muscle diseases.

17. A use of a composition comprising mesenchymal stem cells genetically engineered to secrete a mesenchymal stem cell (MSC) self-maintenance factor (MSMF) or overexpress MSMF compared to parent cells, or comprising MSMF, for preparing a therapeutic agent for muscle diseases.
